(19) Europäisches Patentamt
European Patent Office
Office européen des brevets

(11) Veröffentlichungsnummer: **0 434 982 B1**

# (12) EUROPÄISCHE PATENTSCHRIFT

(45) Veröffentlichungstag der Patentschrift: **15.03.95**

(51) Int. Cl.[6]: **C07D 249/14**, A61K 31/41

(21) Anmeldenummer: **90122351.1**

(22) Anmeldetag: **23.11.90**

## (54) 5-(Amino)-triazolylcarbothioamidderivate, Verfahren zu ihrer Herstellung und diese enthaltende Arzneimittel sowie Verwendung der ersteren.

(30) Priorität: **24.11.89 HU 616389**

(43) Veröffentlichungstag der Anmeldung:
**03.07.91 Patentblatt 91/27**

(45) Bekanntmachung des Hinweises auf die Patenterteilung:
**15.03.95 Patentblatt 95/11**

(84) Benannte Vertragsstaaten:
**AT BE CH DE DK ES FR GB GR IT LI NL SE**

(56) Entgegenhaltungen:
**DD-A- 205 905**
**GB-A- 949 170**
**US-A- 2 352 944**

**JOURNAL OF HETEROCYCLIC CHEMISTRY Bd. 9, Nr. 1, 1972, PROVOH US Seiten 99 - 106; T. HIRATA ET AL.: '5-Azapurines and the Structures of sym-Triazole Intermediates'**

**JOURNAL OF HETEROCYCLIC CHEMISTRY Bd. 24, Nr. 6, 1987, PROVOH US Seiten 1685 - 1695; J. REITER ET AL.: 'On Triazoles. XII. The Carbamoylation and Thiocarbamoylation of 5-Amino-1,2,4-triazoles'**

(73) Patentinhaber: **EGIS GYOGYSZERGYAR**
**Kereszturi ut 30-38**
**H-1106 Budapest (HU)**

(72) Erfinder: **Barkoczy, Jozsef Dr.**
**Szirtes u. 4/b**
**H-1016 Budapest (HU)**
Erfinder: **Reiter, Jozsef Dr.**
**Mihalyfi E. u. 32/b**
**H-1022 Budapest (HU)**
Erfinder: **Pongo, Laszlo Dr.**
**Rakospalotai hatar u. 15**
**H-1161 Budapest (HU)**
Erfinder: **Petocz, Lujza Dr.**
**Rakoczi ter 2**
**H-1084 Budapest (HU)**
Erfinder: **Görgenyi, Frigyes Dr.**
**Szakasits A. u. 60/a**
**H-1115 Budapest (HU)**
Erfinder: **Fekete, Marton Dr.**
**Fö u. 49**
**H-1027 Budapest (HU)**
Erfinder: **Gigler, Gabor**
**Lenin krt. 95**
**H-1067 Budapest (HU)**

EP 0 434 982 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**ARCHIV DER PHARMAZIE Bd. 320, Nr. 2, 1987, WEINHEIM DE Seiten 108 - 114; K. WEGNER ET AL.: 'H2-Antihistaminika. Synthese und H2-antagonistische Aktivität heteroaromatischer (Thio)Carboxamide und Triozol(thi)on-Derivate des Piperidinomethylphenoxypropylamins'**

Erfinder: **Gacsalyi, Istvan**
**Baross u. 67**
**H-1201 Budapest (HU)**
Erfinder: **Gyertyan, Istvan**
**Összefogas u. 9**
**H-1165 Budapest (HU)**

(74) Vertreter: **Beszédes, Stephan G., Dr.**
**Patentanwalt**
**Postfach 11 68**
**D-85201 Dachau (DE)**

## Beschreibung

Die Erfindung betrifft 5-(Amino)-triazolylcarbothioamidderivate zur Verwendung als Arzneimittel, insbesondere mit beruhigender (tranquillanter), antidepressiver und/oder spasmolytischer Wirkung, und die Verwendung von 5-(Amino)-triazolylcarbothioamidderivaten sowie ein Verfahren zu ihrer Herstellung.

Aus der US-PS 2 352 944 sind 1-{3,5 Di-[amino]-1H-1,2,4-triazol-1-yl}-N-{alkyl- oder alkenyl}-carbothioamidderivate, bei welchen die Alkyl- beziehungsweise Alkenylreste am Stickstoffatom der Carbothioamidgruppe nicht substituiert sind, bekannt. Für diese Verbindungen ist ganz allgemein die Verwendung als Pharmazeutika ohne jegliche Spezifizierung angegeben.

Ferner ist in ARCHIV DER PHARMAZIE, Band 320, No. 2, 1987, Seiten 108 bis 114 die Verbindung 5-Amino-3-methylthio-N-[3--(3-piperidinomethyl-phenoxy}-propyl]-1H-1,2,4-triazol-1-carbothioamid beschrieben. Als Wirkung dieser Verbindungen ist die $H_2$-antagonistische Wirksamkeit angegeben.

Weiterhin sind aus der DD-PS 205 905 1-(5-Amino-3-alkylthio-1H-1,2,4-triazol-1-yl)-N-(alkyl- beziehungsweise benzylbeziehungsweise aryl)-carbothioamide bekannt. Eine pharmazeutische Wirkung für die in dieser Druckschrift nur als Ausgangssubstanzen verwendeten Verbindungen ist nicht angegeben.

Außerdem sind in der GB-PS 949 170 1-{5-[Amino]-1H-1,2,4-triazol-1-yl}-N-alkyl}-carbothioamide, bei welchen der Alkylrest am Stickstoffatom der Carbothioamidgruppe nicht substituiert ist, beschrieben. Als Verwendung dieser Verbindungen ist die für Herbizide und Fungizide angegeben, von einer pharmazeutischen Verwendung ist keine Rede.

Aus JOURNAL OF HETEROCYCLIC CHEMISTRY, Band 9, No. 1, 1972, Seiten 99 bis 106 sind die Verbindungen 5-Amino-N-methylthio-1H-1,2,4-triazol-1-carboxamid und 5-Amino-N-benzylthio-1H-1,2,4-triazol-1-carboxamid bekannt. Eine pharmakologische Wirkung ist für diese Verbindungen nicht angegeben.

Ferner sind in JOURNAL OF HETEROCYCLIC CHEMISTRY, Band 24, No. 6, 1987, Seiten 1685 bis 1695 1-{5-[amino]-1H-1,2,4-triazol-1-yl}-carbothioamidderivate, bei welchen die 3-Stellung des Triazolringes nicht substituiert ist oder durch einen Methylthio- oder Äthylthiorest oder durch eine Aminogruppe oder eine Dimethylaminogruppe oder einen Piperidino- oder Morpholinorest substituiert ist, und das Stickstoffatom der Carbothioamidgruppe durch einen Methyl- oder Äthylrest oder einen nicht substituierten Benzylrest oder einen methylsubstituierten Benzylrest substituiert ist, beschrieben. Eine pharmakologische Wirkung ist für diese Verbindungen nicht angegeben.

Der Erfindung liegt die Aufgabe zugrunde, 5-(Amino)-triazolylcarbothioamidderivate mit überlegenen pharmakologischen, insbesondere beruhigenden, antidepressiven und/oder spasmolytischen, Wirkungen zur Verwendung als Arzneimittel sowie ein Verfahren zur Herstellung derselben zu schaffen.

Das Obige wurde überrascbenderweise durch die Erfindung erreicht.

Gegenstand der Erfindung sind 5-(Amino)-triazolylcarbothioamidderivate der allgemeinen Formel

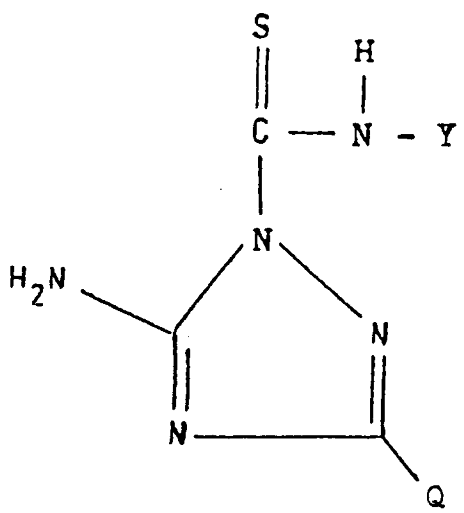

I ,

worin

Q für Wasserstoff, einen, gegebenenfalls durch 1 oder mehr geradkettige[n] und/oder verzweigte[n] Alkylrest(e) mit 1 bis 4 Kohlenstoffatom(en) substituierten, 4 bis 8 gliedrigen, 1 oder mehr Stickstoff- und/oder Sauerstoffatom(e) aufweisenden aromatischen Charakter habenden oder beliebig gesättigten heterocyclischen Rest oder einen Rest der allgemei-

nen Formel

- S - $R_1$ IV

oder

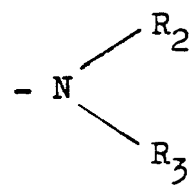

V ,

in welchletzteren

$R_1$ einen geradkettigen oder verzweigten Alkylrest mit 1 bis 6 Kohlenstoffatom(en) darstellt und

$R_2$ und $R_3$ unabhängig voneinander Wasserstoff beziehungsweise geradkettige beziehungsweise verzweigte Alkylreste mit 1 bis 4 Kohlenstoffatom(en) beziehungsweise geradkettige beziehungsweise verzweigte Alkenylreste mit 2 bis 6 Kohlenstoffatomen bedeuten,

steht und

Y einen durch 1 oder mehr Hydroxygruppe(n) oder geradkettige[n] und/oder verzweigte[n] Alkoxyrest(e) mit 1 bis 4 Kohlenstoffatom(en) substituierten Alkylrest mit 1 bis 4 Kohlenstoffatom(en), einen am aromatischen Ring durch 1 oder mehr geradkettige[n] und/oder verzweigte[n] Alkoxyrest(e) mit 1 bis 4 Kohlenstoffatom(en) substituierten Phenylalkylrest mit 1 bis 4 Kohlenstoffatom(en) im geradkettigen oder verzweigten Alkylteil oder einen am Phenylring durch einen durch einen Stickstoff aufweisenden 4- bis 8-gliedrigen aromatischen Charakter habenden oder beliebig gesättigten heterocyclischen Rest substituierten geradkettigen oder verzweigten Alkylrest mit 1 bis 4 Kohlenstoffatom(en) substituierten Phenoxyalkylrest mit 1 bis 4 Kohlenstoffatom(en) im geradkettigen oder verzweigten Alkylteil bedeutet,

mit der weiteren Maßgabe, daß,

a) im Falle daß,

Q für einen Rest der allgemeinen Formel IV,

in welchletzterer

$R_1$ einen Methylrest bedeutet,

steht,

Y von einem Piperidinomethylphenoxypropylrest verschieden ist,

sowie ihre Säureadditionssalze.

Gegenstand der Erfindung ist auch die Verwendung der erfindungsgemäßen Verbindungen unter Einschluß der im obigen Abschnitt a) ausgenommenen Verbindung zur Herstellung von Arzneimitteln mit beruhigenden, antidepressiven und/oder spasmolytischen Wirkungen.

Die Erfindung umfaßt sämtliche isomere und tautomere Formen der 5-(Amino)-triazolylcarbothioamidderivate der allgemeinen Formel I und ihrer Säureadditionssalze.

Beispiele für Alkylreste, gegebenenfalls als Teile von anderen Resten, sind Methyl-, Äthyl-, n-Propyl-, Isopropyl-, n-Butyl, Isobutyl, tert.-Butyl-, n-Pentyl- und n-Hexylreste.

Beispiele für Alkoxyreste, gegebenenfalls als Teile von anderen Resten, sind Methoxy-, Äthoxy-, n-Propoxy-, Isopropoxy-, n-Butoxy-, Isobutoxy- und tert.-Butoxyreste.

Beispiele für Alkenylreste sind Vinyl-, Allyl-, 2-(Methyl)-allyl-, n-Prop-1-enyl-, n-Prop-2-enyl-, n-But-1-enyl-, n-But-2-enyl-, n-Pent-2-enyl- und n-Hex-2-enylreste.

Es ist bevorzugt, daß der beziehungsweise die Alkylrest(e), durch welche[n] der heterocyclische Rest, für den Q stehen kann, substituiert sein kann, der beziehungsweise die Alkylrest(e), für welche[n] $R_2$ und/oder $R_3$ stehen kann beziehungsweise können, der beziehungsweise die Alkoxyrest(e), durch welche[n] der Alkylrest, für welche[n] Y stehen kann, substituiert ist, der Alkylteil des Phenylalkylrestes, für welchen Y stehen kann, der beziehungsweise die Alkoxyrest(e), durch welche[n] der Phenyalkylrest, für welchen Y stehen kann, substituiert ist, beziehungsweise der Alkylrest, durch welchen der Phenoxyalkylrest, für den Y stehen kann, substituiert ist, [ein] solche[r] mit 1 oder 2, insbesondere 1, Kohlenstoffatom(en) ist beziehungsweise sind, beziehungsweise der Alkylteil des Phenoxyalkylrestes, für den Y stehen kann beziehungsweise der Alkylrest, für welchen Y stehen kann, ein solcher mit 2 bis 4, insbesondere 2 oder 3, ganz besonders 3, vor allem ein Propylrest, Kohlenstoffatomen ist.

Ferner ist es bevorzugt, daß der Alkylrest, für den $R_1$ stehen kann, ein solcher mit 1 bis 4, insbesondere 1 oder 2, ganz besonders 1, Kohlenstoffatom(en) ist.

Weiterhin ist es bevorzugt, daß der beziehungweise die Alkenylrest(e), für welche[n] $R_2$ und/oder $R_3$ stehen kann beziehungsweise können, [ein] solche[r] mit 2 bis 4, insbesondere 2 oder 3, ganz besonders 2, Kohlenstoffatomen, vor allem [ein] Allylrest(e), ist beziehungsweise sind.

Außerdem ist es bevorzugt, daß der heterocyclische Rest, für welchen Q stehen kann, und/oder der heterocyclische Rest, durch welchen der Alkylrest, durch den der Phenoxyalkylrest, für den Y stehen kann, substituiert ist, substituiert ist, ein solcher mit 5 oder 6 Ringgliedern, insbesondere ein Piperidyl-, Morpholinyl-, Piperazinyl-, Furyl-, Imidazolyl-, Pyridyl-, Pyrimidinyl-, Pyrrolyl-, Pyrazolyl-, Pyridazinyl-, Isoxazolyl-, Pyrrolinyl-, Pyrrolidinyl-, Imidazolidinyl-, Imidazolinyl-, Pyrazolidinyl-, Pyrazolinyl-, Pyranyl- oder Δ-Piperidinylrest, wie Δ-3-Piperidin-1-ylrest ( Δ-3 bedeutet eine Doppelbindung, ausgehend vom Kohlenstoffatom in der 3-Stellung), ist.

Von den durch durch einen heterocyclischen Rest substituierten Alkylrest substituierten Phenoxyalkylresten, für welche Y stehen kann, sind heterocyclisch substituierte Alkylphenoxypropylreste, insbesondere heterocyclisch substituierte Methylphenoxypropylreste, ganz besonders Piperidinylmethylphenoxypropylreste, vor allem der 3-[3'-(Piperidin-1''-ylmethyl)-phenoxy]-propylrest, bevorzugt.

Von den durch einen Alkylrest substituierten heterocyclischen Resten, für welche Q stehen kann, sind Alkylpiperazinylreste, insbesondere Methylpiperazinylreste, ganz besonders der 4-(Methyl)-piperazinylrest, bevorzugt.

Vorzugsweise ist die Zahl der Alkoxyreste, durch welche der Phenylalkylrest, für den Y stehen kann, substituiert ist, 1 oder 2. Von diesen Resten sind Dialkoxyphenyläthylreste, insbesondere Dimethoxyphenyläthylreste, ganz besonders 3,4-Di-(methoxy)-phenyläthylreste, vor allem der 2-[3',4'-Di-(methoxy)-phenyläthylrest, bevorzugt.

Von den durch eine Hydroxygruppe substituierten Alkylresten, für welche Y stehen kann, sind Hydroxypropyl- und Hydroxyäthylreste, insbesondere der 3-(Hydroxy)-propylrest, der 2-(Hydroxy)-propylrest, der 2-(Hydroxy)-äthylrest und der 2-(Hydroxy)-but-1-ylrest, bevorzugt.

Vorzugsweise ist die Zahl der Alkoxyreste, durch welche der Alkylrest, für den Y stehen kann, substituiert sein kann, 1 oder 2. Von diesen Resten sind Dimethoxyäthylreste, insbesondere der 2,2-Di-(methoxy)-äthylrest, und Methoxyäthylreste, insbesondere der 2-(Methoxy)-äthylrest, bevorzugt.

Eine besonders bevorzugte Untergruppe der erfindungsgemäßen 5-(Amino)-triazolylcarbothioamidderivate sind diejenigen, bei welchen Q einen Morpholinylrest, einen Di-(alkyl)-aminorest, einen Alkylthiorest oder einen 4-(Methyl)-piperazinylrest bedeutet.

Eine andere besonders bevorzugte Untergruppe der erfindungsgemäßen 5-(Amino)-triazolylcarbothioamidderivate sind diejenigen, bei welchen Y einen 3-[3'-(Piperidin-1''-yl-methyl)-phenoxy]-propylrest oder einen am aromatischen Ring durch 1 oder 2 Alkoxyrest(e) substituierten 2-(Phenyl)-äthylrest, insbesondere 2-[3',4'-Di-(methoxy)-phenyl]-äthylrest, bedeutet.

Ganz besonders bevorzugte erfindungsgemäße 5-(Amino)-triazolylcarbothioamidderivate sind

1-{5-[Amino]-3-[morpholino]-1H-1,2,4-triazol-1-yl}-N-{3'-[3''-(piperidin-1'''-yl-methyl)-phenoxy]-propyl}-carbothioamid,

1-{5-[Amino]-3-[dimethylamino]-1H-1,2,4-triazol-1-yl}-N-{3'-[3''-(piperidin-1'''-yl-methyl)-phenoxy]-propyl}-carbothioamid,

1-{5-[Amino]-3-[4'-(methyl)-piperazinyl]-1H-1,2,4-triazol-1-yl}-N-{3'-[3''-(piperidin-1'''-yl-methyl)-phenoxy]-propyl}-carbothioamid und

1-{5-[Amino]-3-[methylthio]-1H-1,2,4-triazol-1-yl}-N-{2'-[3'',4''-di-(methoxy)-phenyl]-äthyl}-carbothioamid.

Die Säureadditionssalze der erfindungsgemäßen 5-(Amino]-triazolylcarbothioamidderivate sind zweckmäßig solche mit therapeutisch brauchbaren anorganischen oder organischen Säuren. Beispiele sind Hydrogenhalogenide, wie Hydrochloride oder Hydrobromide, oder Carbonate, Bicarbonate oder Sulfate bzw. Acetate, Fumarate, Maleate, Citrate, Ascorbinate oder Tartrate.

Gegenstand der Erfindung ist auch ein Verfahren zur Herstellung der erfindungsgemäßen 5-(Amino)-triazolylcarbothioamidderivate, welches dadurch gekennzeichnet ist, daß Triazolyldithioester der allgemeinen Formel

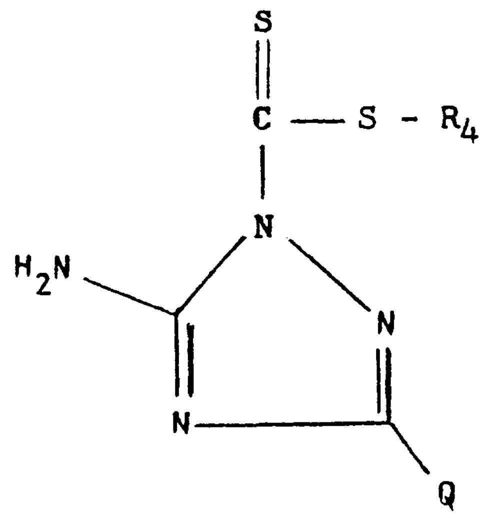

II ,

worin

R4 für einen Alkylrest mit 1 bis 4 Kohlenstoffatom(en) oder einen, gegebenenfalls durch Halogen substituierten, Phenylalkylrest mit 1 bis 4 Kohlenstoffatom(en) im Alkylteil steht und

Q die oben angegebenen Bedeutungen hat, mit Aminen der allgemeinen Formel

$Y—NH_2$ III ,

worin

Y die oben angegebenen Bedeutungen hat, wobei jedoch für die Vertragsstaaten ES und GR Y die in dem Anspruch 1 für diese Vertragsstaaten angegebenen Bedeutungen hat,
umgesetzt werden,
worauf in an sich bekannter Weise gegebenenfalls die erhaltenen 5-(Amino)-triazolylcarbothioamidderivate der allgemeinen Formel I in Säureadditionssalze überführt werden beziehungsweise gegebenenfalls die erhaltenen Säureadditionssalze der 5-(Amino)-triazolylcarbothioamidderivate der allgemeinen Formel I in die freien 5-(Amino)-triazolylcarbothioamidderivate der allgemeinen Formel I oder in andere Säureadditionssalze überführt werden.

Vorzugsweise wird die Umsetzung der Triazolyldithioester der allgemeinen Formel II mit den Aminen der allgemeinen Formel III in inerten organischen Lösungsmitteln durchgeführt. Als Reaktionsmedium können vorteilhaft Alkohole mit 1 bis 4 Kohlenstoffatom(en), die geradkettig oder verzweigt sein können, zum Beispiel Methanol, Äthanol, n-Propanol, Isopropanol, n-Butanol, Isobutanol oder tert.-Butanol, Halogenkohlenwasserstoffe mit 1 oder 2 Kohlenstoffatom(en), zum Beispiel Chloroform, Dichlormethan, 1,2-Di-(chlor)-äthan oder 1,1,2-Tri-(chlor)-äthylen, aromatische Kohlenwasserstoffe, zum Beispiel Benzol, Toluol oder Xylol, Chlorbenzol, Dioxan, Acetonitril oder Dimethylsulfoxyd oder Mischungen dieser Lösungsmittel dienen.

Die Umsetzung wird zweckmäßig bei Temperaturen von 0 bis 160 °C, vorzugsweise 20 bis 120 °C, durchgeführt.

Die Überführung der in Form von Basen erhaltenen 5-(Amino)-triazolylcarbothioamidderivaten der allgemeinen Formel I in ihre Säureadditionssalze kann nach an sich bekannten Verfahrensweisen durchgeführt werden. Diese Salzbildung wird zweckmäßig in der Weise durchgeführt, daß die 5-(Amino)-triazolylcarbothioamidderivatbasen der allgemeinen Formel I in inerten Lösungsmitteln mit den entsprechenden Säuren umgesetzt werden.

Die im erfindungsgemäßen Verfahren als Ausgangssubstanzen verwendeten Triazolyldithioester der allgemeinen Formel II sind bekannte Verbindungen oder können auf einfache Weise hergestellt worden sein (US-PS 3 686 381, DDR-PS 105 897).

Die im erfindungsgemäßen Verfahren als Ausgangssubstanzen verwendeten Amine der allgemeinen Formel III sind Handelsprodukte oder können nach bekannten Verfahren hergestellt worden sein [Houben-Weyl: Methoden der organischen Chemie, Band XI/1, Georg Thieme Verlag, Stuttgart, 1957].

Die erfindungsgemäßen bzw. erfindungsgemäß verwendeten 5-(Amino)-triazolylcarbothioamidderivate haben nämlich bei geringer Toxizität wertvolle pharmazeutische Wirkungen, insbesondere ausgeprägte beruhigende, antidepressive und/oder spasmolytische Wirkungen, bei einigen Verbindungen ergänzt durch

antiphlogistische, analgetische und/oder die Darmperistaltik hemmende Wirkungen.

Die nach der Verfahrensweise von Hoffmeister {Arzneimittel-Forschung (Drug Reasearch) 19 [1969], 846} gemessene, die Tetrabenazin- und Reserpinptose antagonisierende Wirkung von erfindungsgemäßen 5-(Amino)-triazolylcarbothioamidderivaten ist in den unten folgenden Tabellen 1 bzw. 2 zusammengestellt.

Verfahrensweise zur Bestimmung der die Tetrabenazinptose antagonisierenden Wirkung

Der Versuch wurde nach der an Mäuse angepaßten Verfahrensweise von Hoffmeister und Mitarbeitern durchgeführt. Die aus 10 bis 20 Mäusen bestehenden Gruppen wurden mit der zu untersuchenden Verbindung oral behandelt. Die Tiere der Blindversuchs- beziehungsweise Kontrollgruppe wurden ebenfalls oral mit dem Träger behandelt. Nach 30 Minuten wurden 50 mg/kg Tetrabenazin intraperitoneal verabreicht. Die Tiere mit geschlossenem Augenlid (Augenspalte) wurden in jeder Gruppe nach 30, 60, 90 und 120 Minuten gezählt.

Bewertung

Auf Grund sämtlicher Meßwerte wurde die durchschnittliche Ptose in jeder Gruppe berechnet und als Prozentsatz der Abweichung von den bei den Blindversuchs- beziehungsweise Kontrolltieren gemessenen Werten (Hemmung) ausgedrückt. Aus diesen Ergebnissen wurden die $ED_{50}$-Werte berechnet.

Tabelle 1

Die Tetrabenazinptose antagonisierende Wirkung von erfindungsgemäßen 5-(Amino)--triazolylcarbothioamidderivaten an Mäusen

| Untersuchte Verbindung: Bezeichnung | Untersuchte Verbindung: Beispiel | $LD_{50}$-Wert in mg/kg | $ED_{50}$-Wert in mg/kg | Therapeutischer Index |
|---|---|---|---|---|
| 1-{5-[Amino]-3-[4'-(methyl)-piperazinyl]-1H-1,2,4-triazol-1-yl}-N-{3'--[3''-(piperidin-1'''-yl-methyl)--phenoxy]-propyl}-carbothioamid | 3 | über 1 000 | 11 | über 90 |
| 3-{10',11'-Di-(hydro)-5'H-dibenzo[a,d]cyclohepten-5'-yliden}-N,N--di-{methyl}-1-propanamin [Amitriptylin] | Vergleichssubstanz A | 225 | 12 | 18,7 |

Aus der obigen Tabelle 1 geht hervor, daß die Wirkungsbreite der erfindungsgemäßen Verbindung die der Vergleichssubstanz mehrfach übertrifft.

Verfahrensweise zur Bestimmung der Reserpinptose antagonisierenden Wirkung

Die Tiere der aus je 10 Mäusen bestehenden Gruppen werden mit 6 mg/kg Reserpin subkutan behandelt. Nach 60 Minuten wurde die jeweilige zu untersuchende Verbindung den Tieren der Untersuchungsgruppe beziehungsweise der Träger den Tieren der Blindversuchs- beziehungsweise Kontrollgruppe verabreicht. Nach 60 bis 120 Minuten wurden in jeder Gruppe die Tiere mit geschlossenem Augenlid (Augenspalte) gezählt. Die Bewertung erfolgte auf die bei dem vorstehend beschriebenen Ptosis-Prüfversuch beschriebene Weise.

## Tabelle 2

Die Reserpinptose antagonisierende Wirkung von erfindungsgemäßen 5-(Amino)-triazolylcarbothioamidderivaten an Mäusen

| Untersuchte Verbindung: Bezeichnung | Untersuchte Verbindung: Beispiel | $LD_{50}$-Wert in mg/kg | $ED_{50}$-Wert in mg/kg | Therapeutischer Index |
|---|---|---|---|---|
| 1-{5-[Amino]-3-[morpholino]-1H-1,2,4-triazol-1-yl}-N-{3'-[3''-(piperidin-1'''-yl-methyl)-phenoxy]-propyl}-carbothioamid | 1 | über 2 000 | 17 | über 117 |
| 1-{5-[Amino]-3-[methylthio]-1H-1,2,4-triazol-1-yl}-N-{2'-[3'',4''-di-(methoxy)-phenyl]-äthyl}-carbothioamid | 6 | über 2 000 | 20 | über 100 |
| 3-{10',11'-Di-(hydro)-5'H-dibenzo[a,d]cyclohepten-5'-yliden}-N,N-di-{methyl}-1-propanamin [Amitriptylin] | Vergleichssubstanz A | 225 | 63 | 3,6 |

Aus der obigen Tabelle 2 geht hervor, daß die reserpinhemmende Wirkung der erfindungsgemäßen Verbindungen der des als Vergleichssubstanz verwendeten 3-{10',11'-Di-(hydro)-5'H-dibenzo[a,d]-cyclohepten-5'-yliden}-N,N-di-{methyl}-1-propanamines ⟨Amitriptylines⟩ sowohl die absolute Dosis als auch den therapeutischen Index betreffend überlegen sind.

Die den durch 6,7,8,9-Tetra-(hydro)-5H-tetrazoloazepin [Pentetrazol] hervorgerufenen Krampf hemmende Wirkung wurde nach der modifizierten Verfahrensweise von Banzinger und Hane bestimmt (Arch. Int.

Pharmacodyn. 167 [1967], 245) und die Ergebnisse sind der unten folgenden Tabelle 3 zu entnehmen.

Verfahrensweise zur Bestimmung der den durch 6,7,8,9-Tetra-(hydro)-5H-tetrazoloazepin [Pentetrazol] hervorgerufenen Krampf hemmenden Wirkung

Der Versuch wurde nach der modifizierten Verfahrensweise von Banzinger und Hane wie folgt durchgeführt. Die durch 125 mg/kg intraperitoneal verabreichtes 6,7,8,9-Tetra-(hydro)-5H-tetrazoloazepin [Pentetrazol] hervorgerufenen Tonus-Extensor-Krämpfe an den Hintergliedern wurden von Tieren der aus je 6 Mäusen bestehenden Gruppen registriert. Die jeweilige zu untersuchende Verbindung wurde 1 Stunde vor Verabreichung des 6,7,8,9-Tetra-(hydro)-5H-tetrazoloazepines [Pentetrazoles] oral verabreicht. Die Tiere der Blindversuchs- beziehungsweise Kontrollgruppe erhielten den Träger.

Tabelle 3

Die den durch 6,7,8,9-Tetra-(hydro)-5H-tetrazoloazepin [Pentetrazol] hervorgerufenen Krampf antagonisierende Wirkung von erfindungsgemäßen 5-(Amino)-triazolylcarbothioamidderivaten an Mäusen

| Untersuchte Verbindung – Bezeichnung | Untersuchte Verbindung – Beispiel | $LD_{50}$-Wert in mg/kg | $ED_{50}$-Wert in mg/kg | Therapeutischer Index |
|---|---|---|---|---|
| 1-{5-[Amino]-3-[morpholino]-1H-1,2,4-triazol-1-yl}-N-{3'-[3''-(piperidin-1'''-yl-methyl)-phenoxy]-propyl}-carbothioamid | 1 | über 2 000 | 295 | über 6,8 |
| 1-{5-[Amino]-3-[dimethylamino]-1H-1,2,4-triazol-1-yl}-N-{3'-[3''-(piperidin-1'''-yl-methyl)-phenoxy]-propyl}-carbothioamid | 2 | über 1 000 | 64 | über 15,6 |
| 3,5,5-Tri-(methyl)-oxazolidin-2,4-di-(on) [Trimethadion] | Vergleichssubstanz B | 2.050 | 490 | 4,2 |

Aus der obigen Tabelle 3 geht hervor, daß die erfindungsgemäßen Verbindungen die Wirkung der Vergleichssubstanz übertreffen.

Die den durch Nicotin hervorgerufenen Krampf und die Nicotinsterblichkeit hemmende Wirkung wurde nach der Verfahrensweise von Stone bestimmt und die Ergebnisse sind in der weiter unten folgenden Tabelle 4 zusammengestellt.

Verfahrensweise zur Bestimmung der den durch Nicotin hervorgerufenen Krampf und die Nicotinsterblichkeit hemmenden Wirkung

Der Versuch wurde nach der Verfahrensweise von Stone an weißen Mäusen durchgeführt. 1 Stunde nach der oralen Behandlung wurde in die Tiere Nicotin in einer Dosis von 1,4 mg/kg intravenös injiziert. Die Krämpfe und die innerhalb 1 Stunde aufgetretene Sterblichkeit wurden bei den Tieren der behandelten Gruppe und der Blindversuchs- beziehungsweise Kontrollgruppe registriert.

Tabelle 4

Hemmung des durch Nicotin hervorgerufenen Krampfes und der Nicotinsterblichkeit an Mäusen

| Untersuchte Verbindung: Bezeichnung | Untersuchte Verbindung: Beispiel | $LD_{50}$-Wert in mg/kg | $ED_{50}$-Wert in mg/kg | Therapeutischer Index |
|---|---|---|---|---|
| 1-{5-[Amino]-3-[morpholino]-1$\underline{H}$-1,2,4-triazol-1-yl}-N-{3'-[3''-(piperidin-1'''-yl-methyl)-phenoxy]-propyl}-carbothioamid | 1 | über 2 000 | 68 | über 29,4 |
| 1-(Cyclohexyl)-1-(phenyl)-3-(piperidino)-propanol [Trihexyphenidyl] | Vergleichssubstanz C | 365 | 20 | 18,3 |

Aus der obigen Tabelle 4 geht hervor, daß die erfindungsgemäßen Verbindungen eine günstigere therapeutische Breite als die Vergleichsverbindung haben.

Die die 5-[Cyclohex-1'-enyl]-1,5-di-(methyl)-barbitursäure-Narkose {Hexobarbitalnarkose} potenzierende Wirkung wurde nach der Verfahrensweise von Kaergaard (Arch. Int. Pharmacodyn $\underline{2}$ [1967], 170 bestimmt und die gemessenen Werte sind der weiter unten folgenden Tabelle 5 zu entnehmen.

Verfahrensweise zur Bestimmung der die 5-[Cyclohex-1'-enyl]-1,5-di-(methyl)-barbitursäure-Narkose {Hexobarbital-Narkose} potenzierenden Wirkung

Der Versuch wurde an Tieren von aus je 6 Mäusen bestehenden Gruppen durchgeführt. 1 Stunde nach der oralen Verabreichung der jeweiligen zu untersuchenden Verbindung beziehungsweise des Trägers wurde die Narkose durch intravenöse Verabreichung von 40 mg/kg 5-[Cyclohex-1'-enyl]-1,5-di-(methyl)-barbitursäure-Narkose {Hexobarbital} an den Tieren der behandelten Gruppe beziehungsweise der Blindversuchs- beziehungsweise Kontrollgruppe hervorgerufen.

Bewertung

Als eine positive Reaktion zeigend wurden diejenigen Tiere angesehen, deren Schlafzeit das 2,5-fache der der Blindversuchs- beziehungsweise Kontrollgruppe übertraf. Mittels der so transformierten Ergebnisse wurden die $ED_{50}$-Werte berechnet.

Tabelle 5

Die 5-[Cyclohex-1'-enyl]-1,5-di-(methyl)-barbitursäure-Narkose {Hexobarbital-Narkose} potenzierende Wirkung von erfindungsgemäßen 5-(Amino)-triazolylcarbothioamidderivaten bei oraler Verabreichung an Mäusen

| Untersuchte Verbindung: Bezeichnung | Untersuchte Verbindung: Beispiel | $LD_{50}$-Wert in mg/kg | $ED_{50}$-Wert in mg/kg | Therapeutischer Index |
|---|---|---|---|---|
| 1-{5-[Amino]-3-[morpholino]-1H-1,2,4-triazol-1-yl}-N-{3'-[3''-(piperidin-1'''-yl-methyl)-phenoxy]-propyl}-carbothioamid | 1 | über 2 000 | 50 | über 40 |
| 1-{5-[Amino]-3-[dimethylamino]-1H-1,2,4-triazol-1-yl}-N-{3'-[3''-(piperidin-1'''-yl-methyl)-phenoxy]-propyl}-carbothioamid | 2 | über 1 000 | 110 | über 9,1 |
| 1-{5-[Amino]-3-[diallylamino]-1H-1,2,4-triazol-1-yl}-N-{3'-[3''-(piperidin-1'''yl-methyl)-phenoxy]-propyl}-carbothioamid | 5 | über 1 000 | 200 | über 5 |

Fortsetzung der Tabelle 5

| Untersuchte Verbindung | | $LD_{50}$-Wert in mg/kg | $ED_{50}$-Wert in mg/kg | Therapeutischer Index |
|---|---|---|---|---|
| Bezeichnung | Beispiel | | | |
| 2-(Methyl)-2-(propyl)-propan-1,3-dioldicarbamat [Meprobamat] | Vergleichssubstanz D | 1 100 | 270 | 4,1 |

Aus der obigen Tabelle 5 geht hervor, daß die erfindungsgemäßen Verbindungen der Vergleichssubstanz sowohl die absolute Dosis als auch die therapeutische Breite der beruhigenden [tranquillanten] Wirkung betreffend überlegen sind.

Die erfindungsgemäßen bzw. erfindungsgemäß verwendeten Verbindungen können als Arzneimittelpräparate, welche 1 oder mehr dieser Verbindungen als Wirkstoff(e) zusammen mit 1 oder mehr inerten festen

und/oder flüssigen pharmazeutischen Trägerstoff(en) und/oder Verdünnungsmittel(n) und/oder Hilfsstoff(en) enthalten, vorliegen.

Diese Arzneimittel können in Form von Zubereitungen zur oralen Verabreichung, zum Beispiel Tabletten, mit einem Überzug versehenen Tabletten, Dragées, Hart- oder Weichgelatinekapseln, Lösungen, Emulsionen oder Suspensionen zur parenteralen Verabreichung, zum Beispiel injizierbaren Lösungen, oder zur rektalen Verabreichung, zum Beispiel Suppositorien, vorliegen.

Diese Arzneimittel können nach an sich bekannten Verfahrensweisen der pharmazeutischen Technik in der Weise hergestellt werden, daß das beziehungsweise die als Wirkstoff(e) dienende(n) 5-(Amino)-triazolylcarbothioamidderivat(e) mit 1 oder mehr inerten organischen und/oder anorganischen festen und/oder flüssigen Trägerstoff(en), Verdünnungsmittel(n) und/oder Hilfsstoff(en) vermischt und in eine galenische Form gebracht wird beziehungsweise werden.

Die Tabletten, mit einem Überzug versehenen Tabletten, Dragées und Hartgelatinekapseln können als Träger zum Beispiel Milchzucker [Lactose], Maisstärke, Kartoffelstärke, Talk, Magnesiumcarbonat, Magnesiumstearat, Calciumcarbonat und/oder Stearinsäure und/oder [ein] Salz(e) derselben enthalten. Für die Weichgelatinekapseln können als Träger zum Beispiel [ein] Pflanzenöl(e), Fett(e), Wachs(e) und/oder Polyol(e) passender Konsistenz dienen. Die Lösungen und Sirupe können als Träger zum Beispiel Wasser, [ein] Polyol(e), wie Polyäthylenglykol, Rohrzucker (Saccharose] und/oder Glucose enthalten. Für die injizierbaren Lösungen können als Träger zum Beispiel Wasser, [ein] Alkohol(e), [ein] Polyol(e), Glycerin und/oder [ein] Pflanzenöl(e) dienen.

Die Suppositorien können als Träger zum Beispiel [ein] Öl(e), Wachs(e), Fett(e) und/oder Polyol(e) passender Konsistenz enthalten.

Die die erfindungsgemäßen bzw. erfindungsgemäß verwendeten Verbindungen enthaltenden Arzneimittel können auch [ein] Stabilisierungsmittel, zum Beispiel ein Antioxydans oder Antioxydantien enthalten.

Als Hilfsstoff(e) können diese Arzneimittel beispielsweise [ein] Netz-, Emulgier- und/oder Stabilisierungsmittel und/oder [einen] Süßstoff(e), [einen] Aromastoff(e), [ein] Salz(e) zur Veränderung des osmotischen Druckes und/oder [einen] Puffer enthalten.

Die tägliche Dosis der erfindungsgemäBen beziehungsweise erfindungsgemäß verwendeten 5-(Amino)-triazolylcarbothioamidderivate kann innerhalb weiter Grenzen variieren und hängt von mehreren Faktoren, zum Beispiel der Wirksamkeit des Wirkstoffes und dem Alter und Zustand des Patienten, ab. Die tägliche orale Dosis beträgt im allgemeinen 10 bis 10 000 mg, vorzugsweise 50 bis 1 000 mg/Tag. Diese Dosis kann auf einmal oder in mehreren gleichen oder verschiedenen Teilen verabreicht werden. Es sei bemerkt, daß die obigen Werte nur informativen Charakter haben und die anwendbare Wirkstoffdosis immer vom Arzt vorzuschreiben ist.

Die erfindungsgemäßen bzw. erfindungsgemäß verwendeten 5-(Amino)-triazolylcarbothioamidderivate sind vorzugsweise in Form von Präparaten zur oralen Verabreichung zubereitet. Besonders vorteilhaft sind Tabletten oder Kapseln mit einem Wirkstoffgehalt von 250 mg.

Die Erfindung wird an Hand der folgenden Beispiele näher erläutert.

Beispiel 1

1-(5-Amino-3-morpholino-1H-1,2,4-triazol-1-yl)-N-{3-[3-(1-piperidinyl-methyl)-phenoxy]-propyl}-carbothioamid

2,59 g (0,01 Mol) 1-(5-Amino-3-morpholino-1H-1,2,4-triazol-1-yl)-dithiocarbonsäuremethylester werden in 5 ml Dimethylsulfoxyd gelöst, worauf der Lösung 2,48 g (0,01 Mol) 3-[3-(1-Piperidinyl-methyl)-phenoxy]-propylamin unter Wasserkühlung zugegeben werden. Das Reaktionsgemisch wird 8 Stunden lang bei Raumtemperatur gerührt, und in Wasser gegossen. Die ausgeschiedenen Kristalle werden abfiltriert und zuerst aus Acetonitril und danach aus Cyclohexan umkristallisiert. Es werden 2,89 g der im Titel genannten Verbindung erhalten, Ausbeute 63 %, F.: 128-130 °C.

Beispiel 2

1-(5-Amino-3-dimethylamino-1H-1,2,4-triazol-1-yl)-N-{3-[3-(1-piperidinyl-methyl)-phenoxy]-propyl}-carbothioamid

2,17 g (0,01 Mol) 1-(5-Amino-3-dimethylamino-1H-1,2,4-triazol-1-yl)-dithiocarbonsäuremethylester werden in 5 ml Dimethylsulfoxyd gelöst, worauf der Lösung 2,48 g (0,01 Mol) 3-[3-(1-piperidinyl-methyl)-phenoxy]-propylamin unter Wasserkühlung zugegeben werden. Das Reaktionsgemisch wird 8 Stunden lang

bei Raumtemperatur gerührt und in Wasser gegossen. Die ausgeschiedenen Kristalle werden abfiltriert und aus 2-Propanol umkristallisiert. Es werden 2,83 g der im Titel genannten Verbindung erhalten, Ausbeute 68 %, F.: 104-106 °C.

Beispiel 3

1-[5-Amino-3-(4-methyl-piperazinyl)-1H-1,2,4-triazol-1-yl]-N-{3-[3-(1-piperidinyl-methyl)phenoxy]-propyl}-carbothioamid

2,72 g (0,01 Mol) 1-[5-Amino-3-(4-methylpiperazinyl)-1H-1,2,4-triazol-1-yl]-dithiocarbonsäuremethylester werden in 5 ml Dimethylsulfoxyd gelöst, worauf der Lösung 2,48 g (0,01 Mol) 3-[3-(1-piperidinyl-methyl)-phenoxy]-propylamin unter Wasserkühlung zugegeben werden. Das Reaktionsgemisch wird 8 Stunden lang bei Raumtemperatur gerührt, wonach 1 ml Wasser zugetropft wird und nach einstündigem Rühren 10 ml n-Hexan tropfenweise zugegeben werden. Das Gemisch wird eine weitere Stunde lang gerührt, die ausgeschiedenen Kristalle werden abfiltriert und aus 2-Propanol umkristallisiert. Es werden 2,83 g der im Titel genannten Verbindung erhalten, Ausbeute 68 %, F.: 92-93 °C.

Beispiel 4

1-(5-Amino-3-piperidinyl-1H-1,2,4-triazol-1-yl)-N-{3-[3-(1-piperidinyl-methyl)-phenoxy]-propyl}-carbothioamid

2,57 g (0,01 Mol) 1-(5-Amino-3-piperidinyl-1H-1,2,4-triazol-1-yl)-dithiocarbonsäuremethylester werden in 5 ml Dimethylsulfoxyd gelöst, worauf der Lösung 2,48 g (0,01 Mol) 3-[3-(1-piperidinyl-methyl)-phenoxy]-propylamin unter Wasserkühlung zugegeben werden. Das Reaktionsgemisch wird 89 Stunden lang bei Raumtemperatur gerührt, wonach 1 ml Wasser zugetropft und nach einstündigem Rühren 10 ml n-Hexan tropfenweise zugegeben werden. Das Gemisch wird eine weitere Stunde lang gerührt, die ausgeschiedenen Kristalle werden abfiltriert und aus 2-Propanol umkristallisiert. Es werden 3,74 g der im Titel genannten Verbindung erhalten, Ausbeute 82 %, F.: 107-108 °C.

Beispiel 5

1-(5-Amino-3-diallylamino-1H-1,2,4-triazol-1-yl)-N-{3-[3-(1-piperidinyl-methyl)-phenoxy]-propyl}-carbothioamid

2,69 g (0,01 Mol) 1-(5-Amino-3-diallylamino-1H-1,2,4-triazol-1-yl)-dithiocarbonsäuremethylester werden in 5 ml Dimethylsulfoxyd gelöst, worauf der Lösung 2,48 g (0,01 Mol) 3-[3-(1-piperidinyl-methyl)-phenoxy]-propylamin unter Wasserkühlung zugegeben werden. Der Reaktionsgemisch wird 8 Stunden lang bei Raumtemperatur gerührt, wonach 1 ml Wasser zugetropft und nach einstündigem Rühren 50 ml n-Hexan zugetropft werden. Das Gemisch wird eine Stunde lang gerührt, die ausgeschiedenen Kristalle werden abfiltriert und aus 2-Propanol umkristallisiert. Es werden 3,65 g der im Titel genannten Verbindung erhalten, Ausbeute 78 %, F.: 94-96 °C.

Beispiel 6

1-(5-Amino-3-methylthio-1H-1,2,4-triazol-1-yl)-N-[2-(3,4-dimethoxy-phenyl)-äthyl]-carbothioamid

2,20 g (0,01 Mol) 1-(5-Amino-3-methylthio-1H-1,2,4-triazol-1-yl)-dithiocarbonsäuremethylester werden in 10 ml Dimethylsulfoxyd gelöst, worauf der Lösung 1,81 g (0,01 Mol) 2-(3,4-Dimethoxy-phenyl)-äthylamin unter Wasserkühlung zugegeben werden. Das Reaktionsgemisch wird 8 Stunden lang bei Raumtemperatur gerührt, und in 15 ml Wasser gegossen. Die ausgeschiedenen Kristalle werden abfiltriert und aus Äthanol umkristallisiert. Es werden 2,44 g der im Titel genannten Verbindung erhalten, Ausbeute 69 %, F.: 135-137 °C.

Beispiel 7

1-(5-Amino-3-morpholino-1H-1,2,4-triazol-1-yl)-N-[2-(3,4-dimethoxy-phenyl)-äthyl]-carbothioamid

2,59 g (0,01 Mol) 1-(5-Amino-3-morpholino-1H-1,2,4-triazol-1-yl)-dithiocarbonsäuremethylester werden in 10 ml Dimethylsulfoxyd gelöst, worauf der Lösung 1,81 g (0,01 Mol) 2-(3,4-Dimethoxy-phenyl)-äthylamin unter Wasserkühlung zugegeben werden. Das Reaktionsgemisch wird 8 Stunden lang bei Raumtemperatur gerührt und in 15 ml Wasser gegossen. Die ausgeschiedenen Kristalle werden abfiltriert und aus Äthanol umkristallisiert. Es werden 2,66 g der im Titel genannten Verbindung erhalten, Ausbeute 84 %, F.: 142-143 °C.

Beispiel 8

1-(5-Amino-3-morpholino-1H-1,2,4-triazol-1-yl)-N-(3-hydroxypropyl)-carbothioamid

2,59 g (0,01 Mol) 1-(5-Amino-3-morpholino-1H-1,2,4-triazol-1-yl)-dithiocarbonsäuremethylester werden in 25 ml Methanol in Gegenwart von 0,92 ml (0,12 Mol) 3-Aminopropanol unter Rühren eine Stunde lang zum Sieden erhitzt. Das Reaktionsgemisch wird zur Trockne eingedampft und der Rückstand aus Acetonitril umkristallisiert. Es werden 2,70 g der im Titel genannten Verbindung erhalten, Ausbeute 94 %, F.: 116-118 °C.

Beispiel 9

1-(5-Amino-3-morpholino-1H-1,2,4-triazol-1-yl)-N-(3-hydroxyprop-1-yl)-carbothioamid

Man verfährt wie im Beispiel 8 mit dem Unterschied, dass als Lösungsmittel statt Methanol 30 ml Dioxan verwendet werden und das Rohprodukt aus Methanol umkristallisiert wird. Es werden 2,48 g der im Titel genannten Verbindung erhalten, Ausbeute 84 %, F.: 116-118 °C.

Beispiel 10

1-(5-Amino-3-morpholino-1H-1,2,4-triazol-1-yl)-N-(2-hydroxyäthyl)-carbothioamid

2,59 g (0,01 Mol) 1-(5-Amino-3-morpholino-1H-1,2,4-triazol-1-yl)-dithiocarbonsäuremethylester werden in 25 ml Methanol in Gegenwart von 0,72 ml (0,12 Mol) 2-Aminoäthanol eine Stunde lang unter Rühren zum Sieden erhitzt. Dag Reaktionsgemisch wird zur Trockne eingedampft und der Rückstand aus Wasser umkristallisiert. Es werden 2,47 g der im Titel genannten Verbindung erhalten, Ausbeute 91 %, F.: 146-148 °C.

Beispiel 11

1-(5-Amino-3-morpholino-1H-1,2,4-triazol-1-yl)-N-(2-hydroxyäthyl)-carbothioamid

Man verfährt wie im Beispiel 10, mit dem Unterschied, dass man als Lösungsmittel statt Methanol 30 ml Dioxan verwendet und das Rohprodukt aus Methanol umkristallisiert. Es werden 2,34 g der im Titel genannten Verbindung erhalten, Ausbeute 86 %, F.: 146-148 °C.

Beispiel 12

1-(5-Amino-3-dimethylamino-1H-1,2,4-triazol-1-yl)-N-(2-hydroxyäthyl)-carbothioamid

2,17 g (0,01 Mol) 1-(5-Amino-3-dimethylamino-1H-1,2,4-triazol-1-yl)-dithiocarbonsäuremethylester werden in 10 ml Äthanol in Gegenwart von 0,72 ml (0,12 Mol) 2-Aminoäthanol eine Stunde lang unter Rühren zum Sieden erhitzt. Das Reaktionsgemisch wird abgekühlt, die ausgeschiedenen Kristalle werden abfiltriert und aus 2-Propanol umkristallisiert. Es werden 1,89 mg der im Titel genannten Verbindung erhalten, Ausbeute 82 %, F.: 148-150 °C.

Beispiel 13

1-[5-Amino-3-(4-methyl-piperazinyl)-1H-1,2,4-triazol-1-yl]-N-/2-hydroxyäthyl)-carbothioamid

2,72 g (0,01 Mol) 1-[5-Amino-3-(4-methylpiperazinyl)-1H-1,2,4-triazol-1-yl]-dithiocarbonsäuremethylester werden in 10 ml Äthanol in Gegenwart von 0,72 ml (0,12 Mol) 2-Aminoäthanol eine Stunde lang unter Rühren zum Sieden erhitzt. Das Reaktionsgemisch wird abgekühlt, die ausgeschiedenen Kristalle werden abfiltriert und aus 2-Propanol umkristallisiert. Es werden 2,50 g der im Titel genannten Verbindung erhalten, Ausbeute 88 %, F.: 181-183 °C.

Beispiel 14

1-(5-Amino-3-morpholino-1H-1,2,4-triazol-1-yl)-N-(2-hydroxy-prop-1-yl)-carbothioamid

2,59 g (0,01 Mol) 1-(5-Amino-3-morpholino-1H-1,2,4-triazol-1-yl)-dithiocarbonsäuremethylester werden in 30 ml Dioxan in Gegenwart von 0,93 ml (0,12 Mol) 2-Hydroxy-propylamin 2 Stunden lang unter Rühren zum Sieden erhitzt. Das Reaktionsgemisch wird zur Trockne eingedampft und der Rückstand aus Methanol umkristallisiert. Es werden 2,32 g der im Titel genannten Verbindung erhalten, Ausbeute 81 %, F.: 135-137 °C.

Beispiel 15

1-(5-Amino-3-morpholino-1H-1,2,4-triazol-1-yl)-N-(2,2-dimethoxyäthyl)-carbothioamid

2,59 g (0,01 Mol) 1-(5-Amino-3-morpholino-1H-1,2,4-triazol-1-yl)-dithiocarbonsäuremethylester werden in 30 ml Dioxan in Gegenwart von 1,3 ml (0,12 Mol) 2-Amino-acetaldehyd-dimethylacetal 2 Stunden lang unter Rühren zum Sieden erhitzt. Das Reaktionsgemisch wird zur Trockne eingedampft und der Rückstand aus Äthanol umkristallisiert. Es werden 2,37 g der im Titel genannten Verbindung erhalten, Ausbeute 75 %, F.: 134-135 °C.

Beispiel 16

1-(5-Amino-3-morpholino-1H-1,2,4-triazol-1-yl)-N-(2,2-dimethoxyäthyl)-carbothioamid

2,59 g (0,01 Mol) 1-(5-Amino-3-morpholino-1H-1,2,4-triazol-1-yl)-dithiocarbonsäuremethylester werden in 20 ml Methanol in Gegenwart von 1,3 ml (0,12 Mol) 2-Amino-acetaldehyd-dimethylacetal 4 Stunden lang unter Rühren zum Sieden erhitzt. Das Reaktionsgemisch wird abgekühlt, die ausgeschiedenen Kristalle werden abfiltriert und aus Äthanol umkristallisiert. Es werden 2,80 g der im Titel genannten Verbindung erhalten, Ausbeute 88 %, F.: 134-135 °C.

Beispiel 17

1-(5-Amino-3-morpholino-1H-1,2,4-triazol-1-yl)-N-(2-methoxyäthyl)-carbothioamid

2,59 g (0,01 Mol) 1-(5-Amino-3-morpholino-1H-1,2,4-triazol-1-yl)-dithiocarbonsäuremethylester werden in 30 ml Dioxan in Gegenwart von 1,03 ml (0,12 Mol) 2-Methoxyäthylamin 4 Stunden lang unter Rühren zum Sieden erhitzt. Das Reaktionsgemisch wird zur Trockne engedampft und der Rückstand aus 2-Propanol umkristallisiert. Es werden 2,06 g der im Titel genannten Verbindung erhalten, Ausbeute 72 %, F.: 103-105 °C.

Beispiel 18

1-(5-Amino-3-methylthio-1H-1,2,4-triazol-1-yl)-N-(2-hydroxyäthyl)-carbothioamid

2,20 g (0,01 Mol) 1-(5-Amino-3-methylthio-1H-1,2,4-triazol-1-yl)-dithiocarbonsäuremethylester werden in 12 ml Äthanol in Gegenwart von 0,72 ml (0,12 Mol) 2-Aminoäthanol bei Raumtemperatur 12 Stunden lang gerührt. Das Reaktionsgemisch wird unter vermindertem Druck engedampft und der Rückstand aus Acetonitril umkristallisiert. Es werden 0,68 g der im Titel genannten Verbindung erhalten, Ausbeute 29 %,

F.: 131-132 °C.

Beispiel 19

1-(5-Amino-3-morpholino-1H-1,2,4-triazol-1-yl)-N-(2-hydroxy-but-1-yl)-carbothioamid

2,59 g (0,01 Mol) 1-(5-Amino-3-morpholino-1H-1,2,4-triazol-1-yl)-dithiocarbonsäuremethylester werden in 15 ml Dimethylsulfoxyd in Gegenwart von 0,95 ml (0,01 Mol) 2-Aminobutanol bei Raumtemperatur 10 Stunden lang gerührt. Dem Reaktionsgemisch werden 5 g zerstoßenes Eis und 10 ml Wasser zugegeben. Das auskristallisierte Produkt wird abfiltriert und aus Isopropanol umkristallisiert. Es werden 0,69 g der im Titel genannten Verbindung erhalten, Ausbeute 23 %, F.: 133-135 °C.

Beispiel 20

1-(5-Amino-3-methylthio-1H-1,2,4-triazol-1-yl)-N-{3-[3-(1-piperidinyl-methyl)-phenoxy]-propyl}-carbothioamid
(an sich bekannt aus Archiv der Pharmazie, 1987, Seiten 108 - 114)

2,20 g (0,01 Mol) 1-(5-Amino-3-methylthio-1H-1,2,4-triazol-1-yl)-dithiocarbonsäuremethylester werden in 5 ml Dimethylsulfoxyd gelöst, worauf der Lösung unter Wasserkühlung 2,48 g (0,01 Mol) 3-[3-(1-Piperidinyl-methyl)-phenoxy]-propyl-amin zugegeben werden. Das Reaktionsgemisch wird bei Raumtemperatur 5 Stunden lang gerührt. Es findet eine Kristallisation statt. Dem Reaktionsgemisch wird wenig (etwa 1 ml) Wasser zugegeben, das Gemisch wird filtriert und das kristalline Produkt aus Äthanol umkristallisiert. Es werden 1,93 g der im Titel genannten Verbindung erhalten, Ausbeute 46 %, F.: 117-118 °C.

Beispiel 21

1-(5-Amino-1H-1,2,4-triazol-1-yl)-N-{3-[3-(1-piperidinyl-methyl)-phenoxy]-propyl}-carbothioamid

1,74 g (0,01 Mol) 1-(5-Amino-1H-1,2,4-triazol-1-yl)-dithiocarbonsäuremethylester werden in 10 ml Dimethylsulfoxyd gelöst, worauf der Lösung unter Wasserkühlung 2,48 g (0,01 Mol) 3-[3-(1-Piperidinyl-methyl)-phenoxy]-propylamin zugegeben werden. Das Reaktionsgemisch wird bei Raumtemperatur 12 Stunden lang gerührt. Die ausgeschiedenen Kristalle werden abfiltriert und aus Äthanol umkristallisiert. Es werden 1,57 g der im Titel genannten Verbindung erhalten, Ausbeute 42 %, F.: 102-105 °C.

Beispiel 22

Tabletten

Nach an sich bekannten Verfahrensweisen der pharmazeutischen Technik werden Tabletten folgender Zusammensetzung hergestellt:

| Komponente | Menge, mg/Tablette |
|---|---|
| 1-(5-Amino-3-morpholino-1H-1,2,4-triazol-1-yl)-N-{3-[3-(1-piperidinyl-methyl)phenoxy]-propyl}carbothioamid | 250 |
| Milchzucker [Lactose] | 61,8 |
| Stärke | 43,2 |
| Polyvinylpyrrolidon | 22,5 |
| Stearinsäure | 9,0 |
| Talk | 13,5 |
| | Gesamtmenge: 400 mg |

Beispiel 23

Salbe

Nach an sich bekannten Verfahrensweisen der pharmazeutischen Technik wird eine Salbe folgender Zusammensetzung hergestellt:

| Komponente | Menge |
|---|---|
| 1-(5-Amino-3-morpholino-1H-1,2,4-triazol-1-yl)-N-{3-[3-(1-piperidinyl-methyl)phenoxy]-propyl}carbothioamid | 500 mg |
| Unguentum hydrophilicum nonbonicum | 10 000 mg |

Der Wirkstoff ist in der äusseren Phase der Salbe gelöst.

Beispiel 24

Suppositorien

Nach an sich bekannten Verfahrensweisen der pharmazeutischen Technik werden Suppositorien folgender Zusammensetzung hergestellt:

| Komponente | Menge, mg/Suppositorium |
|---|---|
| 1-(5-Amino-3-morpholino-1H-1,2,4-triazol-1-yl)-N-{3-[3-(1-piperidinyl-methyl)phenoxy]-propyl}carbothioamid | 100 |
| Lecithin | 48 |
| Cera alba | 96 |
| Kakaobutter | 1870 |
| Destilliertes Wasser | 386 |
| | Gesamtmenge: 2500 mg |

Beispiel 25

Kapseln

Nach an sich bekannten Verfahrensweisen der pharmazeutischen Technik werden Kapseln folgender Zusammensetzung hergestellt:

| Komponente | Menge, mg/Kapsel |
|---|---|
| 1-(5-Amino-3-morpholino-1H-1,2,4-triazol-1-yl)-N-{3-[3-(1-piperidinyl-methyl)phenoxy]-propyl}carbothioamid | 50 |
| Milchzucker | 119 |
| Stärke | 10 |
| Magnesiumstearat | 1 |
| | Gesamtmenge: 180 mg |

## Patentansprüche

**Patentansprüche für folgende Vertragsstaaten : DE, GB, FR, IT, NL, SE, LI, CH, BE, AT, DK**

**1.** 5-(Amino)-triazolylcarbothioamidderivate der allgemeinen Formel

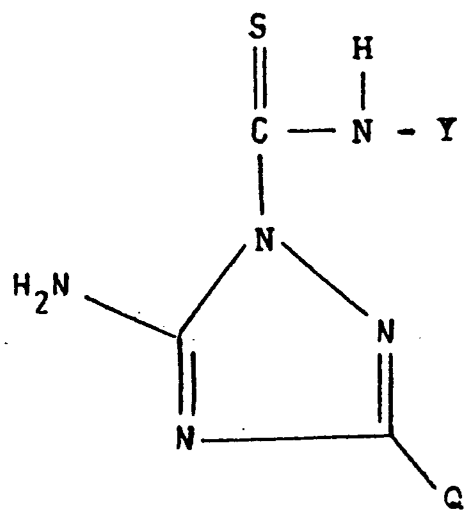

I ,

worin

Q für Wasserstoff, einen, gegebenenfalls durch 1 oder mehr geradkettige[n] und/oder verzweigte[n] Alkylrest(e) mit 1 bis 4 Kohlenstoffatom(en) substituierten, 4 bis 8 gliedrigen, 1 oder mehr Stickstoff- und/oder Saurestoffatom(e) aufweisenden aromatischen Charakter habenden oder beliebig gesättigten heterocyclischen Rest oder einen Rest der allgemeinen Formel

$$- S - R_1 \quad IV$$

oder

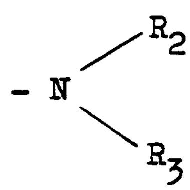

V ,

in welchletzteren

$R_1$ einen geradkettigen oder verzweigten Alkylrest mit 1 bis 6 Kohlenstoffatom(en) darstellt und

$R_2$ und $R_3$ unabhängig voneinander Wasserstoff beziehungsweise geradkettige beziehungsweise verzweigte Alkylreste mit 1 bis 4 Kohlenstoffatom(en) beziehungsweise geradkettige beziehungsweise verzweigte Alkenylreste mit 2 bis 6 Kohlenstoffatomen bedeuten,

steht und

Y einen durch 1 oder mehr Hydroxygruppe(n) oder geradkettige[n] und/oder verzweigte[n] Alkoxyrest(e) mit 1 bis 4 Kohlenstoffatom(en) substituierten Alkylrest mit 1 bis 4 Kohlenstoffatom(en), einen am aromatischen Ring durch 1 oder mehr geradkettige[n] und/oder verzweigte[n] Alkoxyrest(e) mit 1 bis 4 Kohlenstoffatom(en) substituierten Phenylalkylrest mit 1 bis 4 Kohlenstoffatom(en) im geradkettigen oder verzweigten Alkylteil oder einen am Phenylring durch einen durch einen Stickstoff aufweisenden 4- bis 8-gliedrigen aromatischen Charakter habenden oder beliebig gesättigten heterocyclischen Rest substituierten geradkettigen oder verzweigten Alkylrest mit 1 bis 4 Kohlenstoffatom(en) substituierten Phenoxyalkylrest mit 1 bis 4 Kohlenstoffatom(en)

im geradkettigen oder verzweigten Alkylteil bedeutet,
mit der weiteren Maßgabe, daß
a) im Falle daß,
Q für einen Rest der allgemeinen Formel IV,
in welchletzterer
$R_1$ einen Methylrest bedeutet,
steht,
Y von einem Piperidinomethylphenoxypropylrest verschieden ist,
sowie ihre Säureadditionssalze zur Verwendung als Arzneimittel.

**2.** Verwendung der Verbindungen nach Anspruch 1 unter Einschluß der nach Anspruch 1, Abschnitt a) ausgenommenen Verbindung zur Herstellung von Arzneimitteln mit beruhigenden, antidepressiven und/oder spasmolytischen Wirkungen.

**3.** 5-(Amino)-triazolylcarbothioamidderivate nach Anspruch 1 oder nach Anspruch 2 verwendete 5-(Amino)-triazolylcarbothioamidderivate, dadurch gekennzeichnet, daß der beziehungsweise die Alkylrest(e), durch welche[n] der heterocyclische Rest, für den Q stehen kann, substituiert sein kann, der beziehungsweise die Alkylrest(e), für welche[n] $R_2$ und/oder $R_3$ stehen kann beziehungsweise können, der beziehungsweise die Alkoxyrest(e), durch welche[n] der Alkylrest, für welche[n] Y stehen kann, substituiert ist, der Alkylteil des Phenylalkylrestes, für welchen Y stehen kann, der beziehungsweise die Alkoxyrest(e), durch welche[n] der Phenylalkylrest, für welchen Y stehen kann, substituiert ist beziehungsweise der Alkylrest, durch welchen der Phenoxyalkylrest, für den Y stehen kann, substituiert ist, [ein] solche[r] mit 1 oder 2 Kohlenstoffatom(en) ist beziehungsweise sind, beziehungsweise der Alkylteil des Phenoxyalkylrestes, für den Y stehen kann, beziehungsweise der Alkylrest, für welchen Y stehen kann, ein solcher mit 2 bis 4, insbesondere 2 oder 3, Kohlenstoffatomen ist.

**4.** 5-(Amino)-triazolylcarbothioamidderivate nach Anspruch 1 oder 3 oder nach Anspruch 2 verwendete 5-(Amino)-triazolylcarbothioamidderivatedadurch gekennzeichnet, daß der Alkylrest, für den $R_1$ stehen kann, ein solcher mit 1 bis 4, insbesondere 1 oder 2, Kohlenstoffatom(en) ist.

**5.** 5-(Amino)-triazolylcarbothioamidderivate nach Anspruch 1, 3 oder 4 oder nach Anspruch 2 verwendete 5-(Amino)-triazolylcarbothioamidderivate, dadurch gekennzeichnet, daß der beziehungsweise die Alkenylrest(e), für welche[n] $R_2$ und/oder $R_3$ stehen kann beziehungsweise können, [ein] solche[r] mit 2 bis 4, insbesondere 2 oder 3, Kohlenstoffatomen ist beziehungsweise sind.

**6.** 5-(Amino)-triazolylcarbothioamidderivate nach Anspruch 1 oder 3 bis 5 oder nach Anspruch 2 verwendete 5-(Amino)-triazolylcarbothioamidderivate, dadurch gekennzeichnet, daß der heterocyclische Rest, für welchen Q stehen kann, und/oder der heterocyclische Rest, durch welchen der Alkylrest, durch den der Phenoxyalkylrest, für den Y stehen kann, substituiert ist, substituiert ist, ein solcher mit 5 oder 6 Ringgliedern, insbesondere ein Piperidyl-, Morpholinyl-, Piperazinyl-, Furyl-, Imidazolyl-, Pyridyl-, Pyrimidinyl-, Pyrrolyl-, Pyrazolyl-, Pyridazinyl-, Isoxazolyl-, Pyrrolinyl-, Pyrrolidinyl-, Imidazolidinyl-, Imidazolinyl-, Pyrazolidinyl-, Pyrazolinyl-, Pyranyl- oder δ-3-Piperidin-1-ylrest, ist.

**7.** 5-(Amino)-triazolylcarbothioamidderivate nach Anspruch 1, 3, 4 oder 6 oder nach Anspruch 2 verwendete 5-(Amino)-triazolylcarbothioamidderivate, dadurch gekennzeichnet, daß es solche sind, bei welchen Q einen Morpholinylrest, einen Di-(alkyl)-aminorest, einen Alkylthiorest oder einen 4-(Methyl)-piperazinylrest bedeutet.

**8.** 5-(Amino)-triazolylcarbothioamidderivate nach Anspruch 1 oder 3 bis 7 oder nach Anspruch 2 verwendete 5-(Amino)-triazolylcarbothioamidderivate, dadurch gekennzeichnet, daß es solche sind, bei welchen Y einen 3-[3'-(Piperidin-1''-ylmethyl)-phenoxy]-propylrest oder einen am aromatischen Ring durch 1 oder 2 Alkoxyrest(e) substituierten 2-(Phenyl)-äthylrest, insbesondere 2-[3',4'-Di-(methoxy)-phenyl]-äthylrest bedeutet.

**9.** 1-{5-[Amino]-3-[morpholino]-1H-1,2,4-triazol-1-yl}-N--{3'-[3''-(piperidin-1'''-yl-methyl)-phenoxy]-propyl}-carbothioamid, 1-{5-[Amino]-3-[dimethylamino]-1H-1,2,4-triazol-1-yl}-N-{3'-[3''-(piperidin-1'''-yl-methyl)-phenoxy]-propyl}-carbothioamid, 1-{5-[Amino]-3-[4'-(methyl)-piperazinyl]-1H-1,2,4-triazol-1-yl}-N-{3'-[3''-(piperidin-1'''-yl-methyl)phenoxy]-propyl}-carbothioamid und 1-{5-[Amino]-3-[methylthio]-1H-1,2,4-triazol-1-yl}-N-{2'-[3",4"-di-(methoxy)-phenyl]-äthyl}-carbothioamid.

**10.** Verfahren zur Herstellung der 5-(Amino)-triazolylcarbothioamidderivate nach Anspruch 1 oder 3 bis 9 oder nach Anspruch 2 verwendeten 5-(Amino)-triazolylcarbothioamidderivate, dadurch gekennzeichnet, daß man Triazolyldithioester der allgemeinen Formel

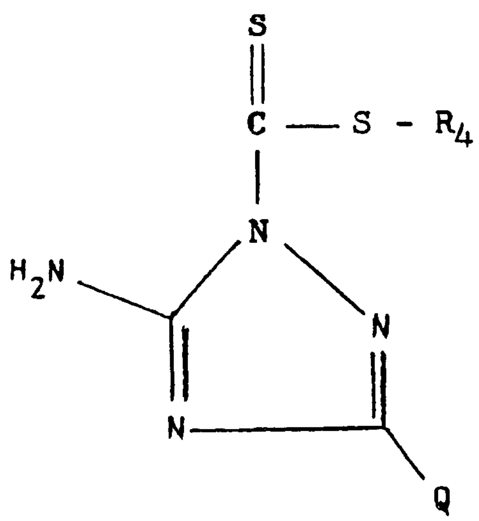

II ,

worin

$R_4$ für einen Alkylrest mit 1 bis 4 Kohlenstoffatom(en) oder einen, gegebenenfalls durch Halogen substituierten, Phenylalkylrest mit 1 bis 4 Kohlenstoffatom(en) im Alkylteil steht und

Q die in den Ansprüchen 1 bis 8 angegebenen Bedeutungen hat,

mit Aminen der allgemeinen Formel

$Y—NH_2$ III ,

worin

Y die in den Ansprüchen 1, 2, 6 oder 8 angegebenen Bedeutungen hat,

umsetzt,

worauf man in an sich bekannter Weise gegebenenfalls die erhaltenen 5-(Amino)-triazolylcarbothioamidderivate der allgemeinen Formel I in Säureadditionssalze überführt beziehungsweise gegebenenfalls die erhaltenen Säureadditionssalze der 5-(Amino)-triazolylcarbothioamidderivate der allgemeinen Formel I in die freien 5-(Amino)-triazolylcarbothioamidderivate der allgemeinen Formel I oder in andere Säureadditionssalze überführt.

**Patentansprüche für folgende Vertragsstaaten : ES, GR**

1. Verfahren zur Herstellung von 5-(Amino)-triazolylcarbothioamidderivaten der allgemeinen Formel

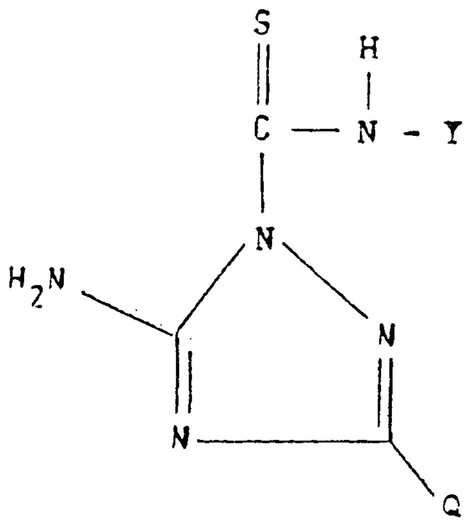

I ,

worin

Q für Wasserstoff, einen, gegebenenfalls durch 1 oder mehr geradkettige[n] und/oder verzweigte[n] Alkylrest(e) mit 1 bis 4 Kohlenstoffatom(en) substituierten, 4 bis 8 gliedrigen, 1 oder mehr Stickstoff- und/oder Sauerstoffatom(e) aufweisenden aromatischen Charakter habenden oder beliebig gesättigten heterocyclischen Rest oder einen Rest der allgemeinen Formel

$- S - R_1$ IV

oder

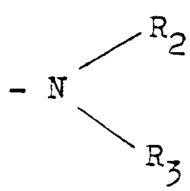

V ,

in welchletzteren

$R_1$ einen geradkettigen oder verzweigten Alkylrest mit 1 bis 6 Kohlenstoffatom(en) darstellt und

$R_2$ und $R_3$ unabhängig voneinander Wasserstoff beziehungsweise geradkettige beziehungsweise verzweigte Alkylreste mit 1 bis 4 Kohlenstoffatom(en) beziehungsweise geradkettige beziehungsweise verzweigte Alkenylreste mit 2 bis 6 Kohlenstoffatomen bedeuten,

steht und

Y einen, gegebenenfalls durch 1 oder mehr Hydroxygruppe(n) oder geradkettige[n] und/oder verzweigte[n] Alkoxyrest(e) mit 1 bis 4 Kohlenstoffatom(en) substituierten, Alkylrest mit 1 bis 4 Kohlenstoffatom(en), einen, gegebenenfalls am aromatischen Ring durch 1 oder mehr geradkettige[n] und/oder verzweigte[n] Alkoxyrest(e) mit 1 bis 4 Kohlenstoffatom(en) substituierten, Phenylalkylrest mit 1 bis 4 Kohlenstoffatom(en) im geradkettigen oder verzweigten Alkylteil oder einen am Phenylring durch einen, gegebenenfalls durch einen Stickstoff aufweisenden 4- bis 8-gliedrigen aromatischen Charakter habenden oder beliebig gesättigten heterocyclischen Rest substituierten, geradkettigen oder verzweigten Alkylrest mit 1 bis 4 Kohlenstoffatom(en)

substituierten Phenoxyalkylrest mit 1 bis 4 Kohlenstoffatom(en) im geradkettigen oder verzweigten Alkylteil bedeutet,

sowie ihren Säureadditionsalzen, dadurch gekennzeichnet, daß man Triazolyldithioester der allgemeinen Formel

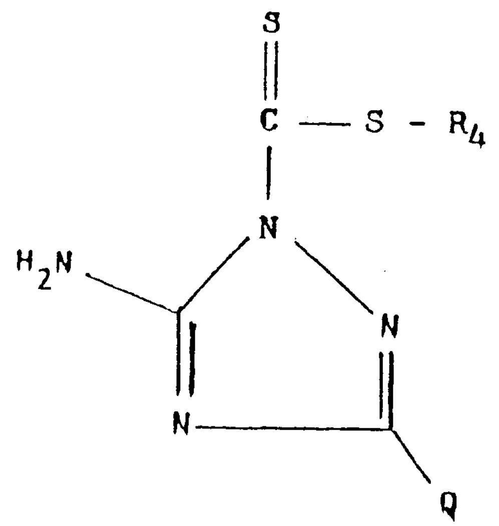

II ,

worin

$R_4$ für einen Alkylrest mit 1 bis 4 Kohlenstoffatom(en) oder einen, gegebenenfalls durch Halogen substituierten, Phenylalkylrest mit 1 bis 4 Kohlenstoffatom(en) im Alkylteil steht und

Q die in den Ansprüchen 1 bis 6 angegebenen Bedeutungen hat,

mit Aminen der allgemeinen Formel

$Y{-}NH_2$ III ,

worin

Y die in den Ansprüchen 1, 2, 5 oder 7 angegebenen Bedeutungen hat,

umsetzt,

worauf man in an sich bekannter Weise gegebenenfalls die erhaltenen 5-(Amino)-triazolylcarbothioamidderivate der allgemeinen Formel I in Säureadditionssalze überführt beziehungsweise gegebenenfalls die erhaltenen Säureadditionssalze der 5-(Amino)-triazolylcarbothioamidderivate der allgemeinen Formel I in die freien 5-(Amino)-triazolylcarbothioamidderivate der allgemeinen Formel I oder in andere Säureadditionssalze überführt.

2. Verfahren nach Anspruch 1, dadurch gekennzeichnet, daß man als 5-(Amino)-triazolylcarbothioamidderivate solche, bei welchen der beziehungsweise die Alkylrest(e), durch welche[n] der heterocyclische Rest, für den Q stehen kann, substituiert sein kann, der beziehungsweise die Alkylrest(e), für welche[n] $R_2$ und/oder $R_3$ stehen kann beziehungsweise können, der beziehungsweise die Alkoxyrest(e), durch welche[n] der Alkylrest, für welche[n] Y stehen kann, substituiert sein kann, der Alkylteil des Phenylalkylrestes, für welchen Y stehen kann, der beziehungsweise die Alkoxyrest(e), durch welche[n] der Phenylalkylrest, für welchen Y stehen kann, substituiert sein kann beziehungsweise der Alkylrest, durch welchen der Phenoxyalkylrest, für den Y stehen kann, substituiert ist, [ein] solche[r] mit 1 oder 2 Kohlenstoffatom(en) ist beziehungsweise sind, beziehungsweise der Alkylteil des Phenoxyalkylrestes, für den Y stehen kann, beziehungsweise der Alkylrest, für welchen Y stehen kann, ein solcher mit 2 bis 4, insbesondere 2 oder 3, Kohlenstoffatomen ist, herstellt.

3. Verfahren nach Anspruch 1 oder 2, dadurch gekennzeichnet, daß man als 5-(Amino)-triazolylcarbothioamidderivate solche, bei welchen der Alkylrest, für den $R_1$ stehen kann, ein solcher mit 1 bis 4, insbesondere 1 oder 2, Kohlenstoffatom(en) ist, herstellt.

4. Verfahren nach Anspruch 1 bis 3, dadurch gekennzeichnet, daß man als 5-(Amino)-triazolylcarbothioamidderivate solche, bei welchen der beziehungsweise die Alkenylrest(e), für welche[n] $R_2$ und/oder $R_3$ stehen kann beziehungsweise können, [ein] solche[r] mit 2 bis 4, insbesondere 2 oder 3, Kohlenstoffatomen ist beziehungsweise sind, herstellt.

5. Verfahren nach Anspruch 1 bis 4, dadurch gekennzeichnet, daß man als 5-(Amino)-triazolylcarbothioamidderivate solche, bei welchen der heterocyclische Rest, für welchen Q stehen kann, und/oder der heterocyclische Rest, durch welchen der Alkylrest, durch den der Phenoxyalkylrest, für den Y stehen kann, substituiert sein kann, substituiert sein kann, ein solcher mit 5 oder 6 Ringgliedern, insbesondere ein Piperidyl-, Morpholinyl-, Piperazinyl-, Furyl-, Imidazolyl-, Pyridyl-, Pyrimidinyl-, Pyrrolyl-, Pyrazolyl-, Pyridyzinyl-, Isoxazolyl-, Pyrrolinyl-, Pyrrolidinyl-, Imidazolidinyl-, Imidazolinyl-, Pyrazolidinyl-, Pyrazolinyl-, Pyranyl- oder δ-3-Piperidin-1-ylrest, ist, herstellt.

6. Verfahren nach Anspruch 1 bis 3 oder 5, dadurch gekennzeichnet, daß man als 5-(Amino)-triazolylcarbothioamidderivate solche, bei welchen Q einen Morpholinylrest, einen Di-(alkyl)-aminorest, einen Alkylthiorest oder einen 4-(Methyl)-piperazinylrest bedeutet, verwendet.

7. Verfahren nach Anspruch 1 bis 6, dadurch gekennzeichnet, daß man als 5-(Amino)-triazolylcarbothioamidderivate solche, bei welchen Y einen 3-[3'-Piperidin-1''-ylmethyl)-phenoxy]-propylrest oder einen am aromatischen Ring durch 1 oder 2 Alkoxyrest(e) substituierten 2-(Phenyl)-äthylrest, insbesondere 2-[3',4'-Di-(methoxy)-phenyl]-äthylrest bedeutet, herstellt.

8. Verfahren nach Anspruch 1 bis 3 oder 5 bis 7, dadurch gekennzeichnet, daß man als 5-(Amino)-triazolylcarbothioamidderivate
1-{5-[Amino]-3-[morpholino]-1H-1,2,4-triazol-1-yl}-N-{3'-[3''-(piperidin-1'''-yl-methyl)-phenoxy]-propyl}-carbothioamid,
1-{5-[Amino]-3-[dimethylamino]-1H-1,2,4-triazol-1-yl}-N-{3'-[3''-(piperidin-1'''-yl-methyl)-phenoxy]-propyl}-carbothioamid,
1-{5-[Amino]-3-[4'-(methyl)-piperazinyl]-1H-1,2,4-triazol-1-yl}-N-{3'-[3''-(piperidin-1'''-yl-methyl)-phenoxy]-propyl}-carbothioamid oder
1-{5-[Amino]-3-[methylthio]-1H-1,2,4-triazol-1-yl}-N-{2'-[3'',4''-di-(methoxy)-phenyl]-äthyl}-carbothioamid herstellt.

**Claims**

**Claims for the following Contracting States : DE, GB, FR, IT, NL, SE, LI, CH, BE, AT, DK**

1. 5-(Amino)-triazolylcarbothioamide derivatives of general formula (I),

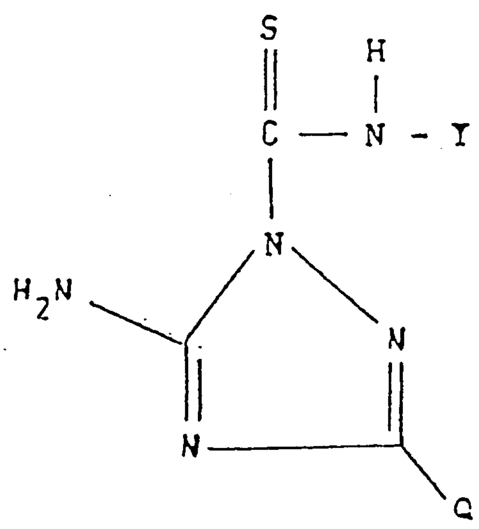

(I)

wherein

Q stands for hydrogen atom, a heterocyclic group of aromatic character or of optional saturation, having 4 to 8 members and containing within the ring 1 or more nitrogen and/or oxygen atom(s), the said group being optionally substituted by 1 or more straight and/or branched chain alkyl group(s) having 1 to 4 carbon atom(s); or a group of general formula

$-S-R_1$ (IV)

or

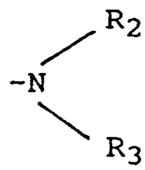

(V)

wherein

$R_1$ stands for straight or branched chain alkyl group with 1-6 carbon atom(s) and

$R_2$ and $R_3$ stand independently from each other for hydrogen atom or straight or branched chain alkyl groups having 1 to 4 carbon atom(s) or for straight or branched chain alkenyl groups having 2 to 6 carbon atoms, and

Y stands for an alkyl group having 1 to 4 carbon atom(s) substituted by 1 or more hydroxy group(s) or straight and/or branched chain alkoxy group(s) having 1 to 4 carbon atom(s); a phenylalkyl group having 1 to 4 carbon atom(s) in the straight or branched chain alkyl moiety, the said phenylalkyl group having 1 or more straight and/or branched chain alkoxy group(s) having 1 to 4 carbon atom(s); or a phenoxy alkyl group having 1 to 4 carbon atom(s) in the straight or branched chain alkyl moiety, this phenoxy alkyl group being substituted on the phenyl ring by a straight or branched chain alkyl group with 1 to 4 carbon atom(s), the latter alkyl group being substituted by an optionally unsaturated heterocyclic group having 4 to 8 ring-member atoms and in the ring having also a nitrogen atom,

with the further proviso that

a) if Q stands for a group of general formula (IV), wherein $R_1$ is a methyl group, then Y is different from piperidino-methylphenoxypropyl group,

and the acid-addition salts for use as medicines.

**2.** Use of the compounds of claim 1, including the compound excluded by point a) of claim 1, for producing medicines having tranquillant, antidepressive and/or spasmolytic effects.

**3.** 5-(Amino)-triazolylcarbothioamide derivatives as claimed in claim 1 or 5-(amino)-triazolylcarbothioamide derivatives used according to claim 2, **characterized** in that the alkyl group(s) serving as the substituent(s) for the heterocyclic group Q; the alkyl group(s) $R_2$ and/or $R_3$; the alkoxy group(s) serving as substituent for the alkyl group Y; the alkyl moiety of the phenylalkyl group Y; the alkoxy group(s) serving as substituent for the phenylalkyl group Y; the alkyl group serving as substituent for the phenoxyalkyl group Y, has 1 or 2 carbon atom(s) and, resp., the alkyl moiety of the phenoxyalkylgroup Y and the alkyl group Y have 2 to 4, preferably 2 to 3, carbon atoms.

**4.** 5-(Amino)-triazolylcarbothioamide derivatives as claimed in claims 1 or 3 or 5-(amino)-triazolylcarbothioamide derivatives as used according to claim 2, **characterized** in that the alkyl group $R_1$ has 1 to 4, preferably 1 or 2, carbon atom(s).

**5.** 5-(Amino)-triazolylcarbothiamide derivatives as claimed in claim 1, 3 or 4 or 5-(amino)-triazolylcarbothioamide derivatives as used according to claim 2, **characterized** in that the alkenyl group(s) $R_2$ and/or $R_3$ have 2 to 4, especially 2 or 3, carbon atoms.

6. 5-(Amino)-triazolylcarbothiamide derivatives as claimed in claims 1 or 3 to 5 or 5-(amino)-triazolylcarbothioamide derivatives as used according to claim 2, **characterized** in that the heterocyclic group Q and/or the heterocyclic group serving as substituent for the alkyl group being substituent of the phenoxyalkyl group Y, have 5 or 6 ring members, preferably piperidyl, morpholinyl, piperazinyl, furyl, imidazolyl, pyridyl, pyrimidinyl, pyrrolyl, pyrazolyl, pyridazinyl, isoxazolyl, pyrrolinyl, pyrrolidinyl, imidazolidinyl, imidazolinyl, pyrazolidinyl, pyrazolinyl, pyranyl or δ-3-piperidin-1-yl group.

7. 5-(Amino)-triazolylcarbothiamide derivatives as claimed in claims 1, 3, 4 or 6 or 5-(amino)-triazolylcarbothioamide derivatives as used according to claim 2, **characterized** in that Q stands for a morpholinyl group, a di(alkyl)-amino group, an alkylthio group or a 4-(methyl)-piperazinyl group.

8. 5-(Amino)-triazolylcarbothiamide derivatives as claimed in claims 1 or 3 to 7 or 5-(amino)-triazolylcarbothioamide derivatives as used according to claim 2, **characterized** in that Y stands for a 3-[3'-(piperidin-1''-yl-methyl)-phenoxy]-propyl group or a 2-(phenyl)-ethyl group substituted on the aromatic ring by 1 or 2 alkoxy group(s), preferably a 2-[3',4'-di(methoxy)-phenyl]-ethyl group.

9. 1-{5-[Amino]-3-[morpholino]-1H-1,2,4-triazol-1-yl}-N--{3'-[3''-(piperidin-1'''-yl-methyl)-phenoxy'-propyl}-carbothioamide,
1-{5-[Amino]-3-[dimethylamino]-1H-1,2,4-triazol-1-yl}-N-{3'--[3''-(piperidin-1'''-yl-methyl)-phenoxy]-propyl}-carbothioamide,
1-{5-[Amino]-3-[4'-(methyl)-piperazinyl]-1H-1,2,4-triazol-1--yl}-N-{3'-[3''-(piperidin-1'''-yl-methyl)-phenoxy'-propyl}-carbothioamide
and
1-{5-[Amino]-3-[methylthio]-1H-1,2,4-triazol-1-yl}-N-{2'-[3'',4''-di-(methoxy)-phenyl]-ethyl}-carbothioamide.

10. A process for preparing 5-(amino)-triazolylcarbothioamide derivatives as claimed in claims 1 or 3 to 9 or 5-(amino)-triazolylcarbothioamidederivatives as used according to claim 2 **characterized** in that a triazolyldithioester of general formula (II)

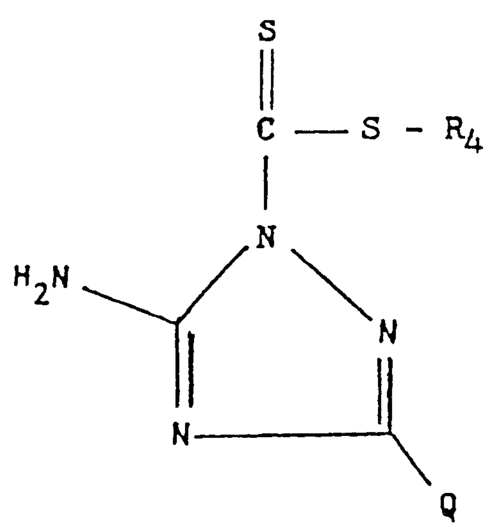

(II)

wherein

$R_4$ stands for an alkyl group having 1 to 4 carbon atom(s) or a phenylalkyl group having 1 to 4 carbon atom(s) in the alkyl moiety and optionally substituted by a halogen atom and

Q has the meaning as given in claims 1 to 8,

is reacted with amines of general formula (III),

$Y\text{-}NH_2$ (III)

wherein

Y has the meanings as given in claims 1, 2, 6 or 8, whereafter the obtained 5-(amino)-triazolylcarbothioamide derivative of general formula (I) is optionally transformed in a way known *per se* to an acid-addition salt or the obtained acid-addition salts of the 5-(amino)-triazolylcarbothioamide derivative of general formula (I) are optionally transformed to the free 5-(amino)-triazolylcarbothioamide derivatives of general formula (I) or to other acid-addition salts.

**Claims for the following Contracting States : ES, GR**

**1.** A process for preparing 5-(amino)-triazolylcarbothioamide derivatives of general formula (I),

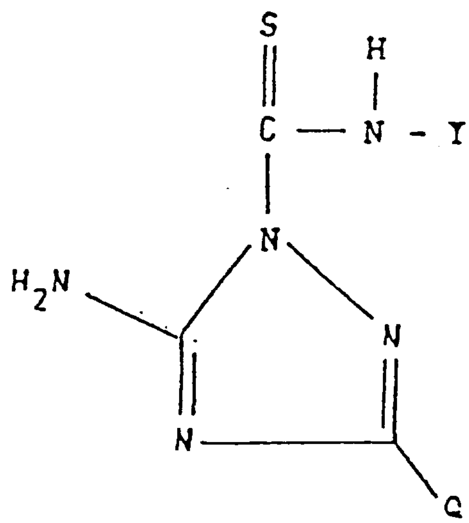

(I)

wherein

Q stands for hydrogen atom, a heterocyclic group of aromatic character or of optional saturation, having 4 to 8 members and containing within the ring 1 or more nitrogen and/or oxygen atom(s), the said group being optionally substituted by 1 or more straight and/or branched chain alkyl group(s) having 1 to 4 carbon atom(s); or a group of general formula

$-S-R_1$ (IV)

or

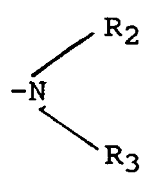

(V)

wherein

$R_1$ stands for straight or branched chain alkyl group with 1-6 carbon atom(s) and

$R_2$ and $R_3$ stand independently from each other for hydrogen atom or straight or branched chain alkyl groups having 1 to 4 carbon atom(s) or for straight or branched chain alkenyl groups having 2 to 6 carbon atoms, and

Y stands for an alkyl group having 1 to 4 carbon atom(s) substituted by 1 or more hydroxy group(s) or straight and/or branched chain alkoxy group(s) having 1 to 4 carbon atom(s); a phenylalkyl group having 1 to 4 carbon atom(s) in the straight or branched chain alkyl moiety, the said phenylalkyl group having 1 or more straight and/or branched chain alkoxy group(s) having 1 to 4 carbon atom(s); or a phenoxy alkyl group having 1 to 4 carbon atom(s) in the straight or branched chain alkyl

moiety, this phenoxy alkyl group being substituted on the phenyl ring by a straight or branched chain alkyl group with 1 to 4 carbon atom(s), the latter alkyl group being substituted by an optionally unsaturated heterocyclic group having 4 to 8 ring-member atoms and in the ring having also a nitrogen atom,

and their acid-addition salts, **characterized** in that a triazolyldithioester of general formula (II)

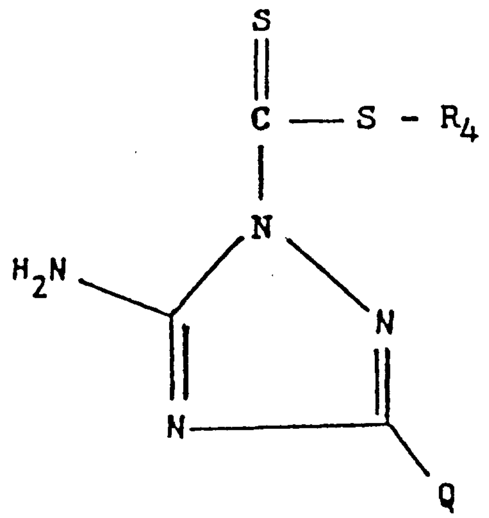

(II)

wherein

$R_4$ stands for an alkyl group having 1 to 4 carbon atom(s) or a phenylalkyl group having 1 to 4 carbon atom(s) in the alkyl moiety and optionally substituted by a halogen atom and

Q has the meaning as given in claims 1 to 8,

is reacted with amines of general formula (III),

$Y\text{-}NH_2$ (III)

wherein

Y has the meanings as given in claims 1, 2, 6 or 8, whereafter the obtained 5-(amino)-triazolylcarbothioamide derivative of general formula (I) is optionally transformed in a way known *per se* to an acid-addition salt or the obtained acid-addition salts of the 5-(amino)-triazolylcarbothioamide derivative of general formula (I) are optionally transformed to the free 5-(amino)-triazolylcarbothioamide derivatives of general formula (I) or to other acid-addition salts.

2. A process as claimed in claim 1, **characterized** in that 5-(amino)-triazolylcarbothioamide derivatives are prepared in which the alkyl group(s) serving as the substituent(s) for the heterocyclic group Q; the alkyl group(s) $R_2$ and/or $R_3$; the alkoxy group(s) serving as possible substituent for the alkyl group Y; the alkyl moiety of the phenylalkyl group Y; the alkoxy group(s) serving as substituent for the phenylalkyl group Y; the alkyl group serving as substituent for the phenoxyalkyl group Y, has 1 or 2 carbon atom(s) and, resp., the alkyl moiety of the phenoxyalkylgroup Y and the alkyl group Y have 2 to 4, preferably 2 to 3, carbon atoms.

3. A process as claimed in claim 1 or 2, **characterized** in that 5-(amino)-triazolylcarbothioamide derivatives are prepared in which the alkyl group $R_1$ has 1 to 4, preferably 1 or 2, carbon atom(s).

4. A process as claimed in claims 1 to 3, **characterized** in that 5-(amino)-triazolylcarbothioamide derivatives are prepared in which the alkenyl group(s) $R_2$ and/or $R_3$ have 2 to 4, especially 2 or 3, carbon atoms.

5. A process as claimed in claims 1 to 4, **characterized** in that 5-(amino)-triazolylcarbothioamide derivatives are prepared in which the heterocyclic group Q and/or the heterocyclic group serving as substituent for the alkyl group being substituent of the phenoxyalkyl group Y, have 5 or 6 ring members, preferably piperidyl, morpholinyl, piperazinyl, furyl, imidazolyl, pyridyl, pyrimidinyl, pyrrolyl,

pyrazolyl, pyridazinyl, isoxazolyl, pyrrolinyl, pyrrolidinyl, imidazolidinyl, imidazolinyl, pyrazolidinyl, pyrazolinyl, pyranyl or δ-3-piperidin-1-yl group.

**6.** A process as claimed in claims 1 to 3 or 5, **characterized** in that 5-(amino)-triazolylcarbothioamide derivatives are prepared in which Q stands for a morpholinyl group, a di(alkyl)-amino group, an alkylthio group or a 4-(methyl)-piperazinyl group.

**7.** A process as claimed in claims 1 to 6, 5-(amino)-triazolylcarbothioamide derivatives are prepared in which Y stands for a 3-[3'-(piperidin-1''yl-methyl)-phenoxy]-propyl group or a 2-(phenyl)-ethyl group substituted on the aromatic ring by 1 or 2 alkoxy group(s), preferably a 2-[3',4'-di(methoxy)-phenyl]-ethyl group.

**8.** A process as claimed in claims 1 to 3 or 5 to 7, **characterized** in that the following 5-(amino)-triazolylcarbothioamide derivatives are prepared:
1-{5-[Amino]-3-[morpholino]-1H-1,2,4-triazol-1-yl}-N-{3'-[3''-(piperidin-1'''-yl-methyl)-phenoxy'-propyl}-carbothioamide,
1-{5-[Amino]-3-[dimethylamino]-1H-1,2,4-triazol-1-yl}-N-{3'-[3''-(piperidin-1'''-yl-methyl)-phenoxy]-propyl}-carbothioamide,
1-{5-[Amino]-3-[4'-(methyl)-piperazinyl]-1H-1,2,4-triazol-1-yl}-N-{3'-[3''-(piperidin-1'''-yl-methyl)-phenoxy'-propyl}-carbothioamide
and
1-{5-[Amino]-3-[methylthio]-1H-1,2,4-triazol-1-yl}-N-{2'-[3'',4''-di-(methoxy)-phenyl]-ethyl}-carbothioamide.

**Revendications**
**Revendications pour les Etats contractants suivants : DE, GB, FR, IT, NL, SE, LI, CH, BE, AT, DK**

**1.** Dérivés de 5-(amino)-triazolylcarbothioamides répondant à la formule générale :

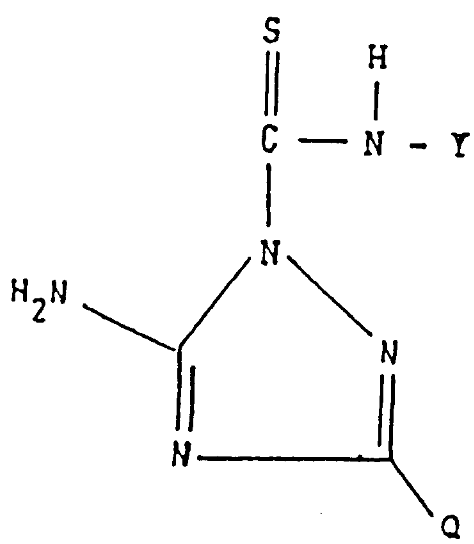

I

dans lesquelles

Q représente un atome d'hydrogène, un reste hétérocyclique éventuellement substitué par 1 ou plusieurs restes alkyles linéaires et/ou ramifiés, ayant de 1 à 4 atomes de carbone, comportant de 4 à 8 chaînons, présentant 1 ou plusieurs atomes d'azote et/ou d'oxygène, ayant un caractère aromatique ou étant saturé d'une manière quelconque, ou un reste répondant à la formule :

$-S-R_1$ IV

ou

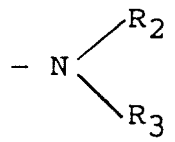

V

dans laquelle

$R_1$ représente un reste alkyle linéaire ou ramifié comportant de 1 à 6 atomes de carbone, et

$R_2$ et $R_3$ signifient, indépendamment l'un de l'autre, un atome d'hydrogène ou des restes alkyles linéaires ou ramifiés comportant de 1 à 4 atomes de carbone, ou des restes alkényles linéaires ou ramifiés comportant de 2 à 6 atomes de carbone, et

Y signifie un reste alkyle comportant de 1 à 4 atomes de carbone, substitué par 1 ou plusieurs groupes hydroxy ou par un ou plusieurs restes alcoxy linéaires et/ou ramifiés comportant de 1 à 4 atomes de carbone, un reste phénylalkyle comportant de 1 à 4 atomes de carbone dans la partie alkyle linéaire ou ramifié, substitué dans le cycle aromatique par 1 ou plusieurs restes alcoxy linéaires et/ou ramifiés comportant de 1 à 4 atomes de carbone, ou un reste phénoxyalkyle comportant de 1 à 4 atomes de carbone dans la partie alkyle linéaire ou ramifié, substitué dans le cycle phénylique par un reste alkyle linéaire ou ramifié comportant de 1 à 4 atomes de carbone, substitué par un reste hétérocyclique comportant un atome d'azote, ayant de 4 à 8 chaînons, présentant un caractère aromatique ou étant saturé d'une manière quelconque,

avec la condition supplémentaire que

a) pour le cas où

Q signifie un reste répondant à la formule générale IV, dans laquelle $R_1$ signifie un reste méthyle,

Y est différent d'un reste pipéridinométhylphénoxypropyle,

ainsi que leurs sels d'addition d'acides, pour une utilisation sous la forme d'un médicament.

**2.** Utilisation des composés selon la revendication 1, y compris le composé exclu selon la revendication 1, paragraphe a), pour la préparation de médicaments ayant des effets apaisants, antidépresseurs et/ou spasmolytiques.

**3.** Dérivés de 5-(amino)-triazolylcarbothioamides selon la revendication 1 ou dérivés de 5-(amino)-triazolylcarbothioamides utilisés selon la revendication 2, caractérisés en ce que le ou les restes alkyles au moyen duquel/desquels le reste hétérocyclique, qui peut être représenté par Q, peut être substitué, le ou les restes alkyles qui peuvent être représentés par $R_2$ et/ou $R_3$, le ou les restes alcoxy, au moyen duquel/desquels le reste alkyle est substitué, qui peut être représenté par Y, la partie alkyle du reste phénylalkyle, qui peut être représentée par Y, le ou les restes alcoxy, au moyen duquel/desquels le reste phénylalkyle est substitué, qui peut être représenté par Y, ou le reste alkyle au moyen duquel le reste phénoxyalkyle est substitué, qui peut être représenté par Y, comporte(nt) 1 ou 2 atomes de carbone, ou la partie alkyle du reste phénoxyalkyle, qui peut être représentée par Y, ou le reste alkyle pouvant être représenté par Y, sont des restes alkyles comportant de 2 à 4, en particulier 2 ou 3 atomes de carbone.

**4.** Dérivés de 5-(amino)-triazolylcarbothioamides selon la revendication 1 ou 3, ou dérivés de 5-(amino)-triazolylcarbothioamidesutilisés selon la revendication 2, caractérisés en ce que le reste alkyle qui peut être représenté par $R_1$ comporte de 1 à 4, en particulier 1 ou 2 atomes de carbone.

**5.** Dérivés de 5-(amino)-triazolylcarbothioamides selon la revendication 1, 3 ou 4, ou dérivés de 5-(amino)-triazolylcarbothioamides utilisés selon la revendication 2, caractérisés en ce que le ou les restes alkényles, qui peuvent être représentés par $R_2$ et/ou $R_3$, comportent de 2 à 4, en particulier 2 ou 3 atomes de carbone.

**6.** Dérivés de 5-(amino)-triazolylcarbothioamides selon la revendication 1 ou l'une des revendications 3 à 5, ou dérivés de 5-(amino)-triazolylcarbothioamides utilisés selon la revendication 2, caractérisés en ce que le reste hétérocyclique qui peut être représenté par Q, et/ou le reste hétérocyclique qui est un

substituant pour le reste alkyle qui est un substituant pour le reste phénoxyalkyle qui peut être représenté par Y, est un reste à 5 ou 6 chaînons, en particulier un reste pipéridyle, morpholinyle, pipérazinyle, furyle, imidazolyle, pyridyle, pyrimidinyle, pyrrolyle, pyrazolyle, pyridazinyle, isoxazolyle, pyrrolinyle, pyrrolidinyle, imidazolidinyle, imidazolinyle, pyrazolidinyle, pyrazolinyle, pyranyle ou δ-3-pipéridine-1-yle.

**7.** Dérivés de 5-(amino)-triazolylcarbothioamides selon la revendication 1, 3, 4 ou 6, ou dérivés de 5-(amino)-triazolylcarbothioamides utilisés selon la revendication 2, caractérisés en ce qu'il s'agit de dérivés dans lesquels Q signifie un reste morpholinyle, un reste di-(alkyl)-amino, un reste alkylthio ou un reste 4-(méthyl)-pipérazinyle.

**8.** Dérivés de 5-(amino)-triazolylcarbothioamides selon la revendication 1 ou l'une des revendications 3 à 7, ou dérivés de 5-(amino)-triazolylcarbothioamides utilisés selon la revendication 2, caractérisés en ce qu'il s'agit de dérivés dans lesquels Y signifie un reste 3-[3'-(pipéridine-1''-ylméthyl)-phénoxy]-propyle ou un reste 2-(phényl)-éthyle substitué dans le cycle aromatique par 1 ou 2 restes alcoxy, en particulier le reste 2-[3',4'-di-(méthoxy)-phényl]-éthyle.

**9.** 1-{5-[amino]-3-[morpholino]-1H-1,2,4-triazol-1-yl}-N-{3'-[3''-(pipéridine-1'''-yl-méthyl)-phénoxy]-propyl)-carbothioamide,
1-{5-[amino]-3-[diméthylamino]-1H-1,2,4-triazol-1-yl)-N-{3'-[3''-(pipéridine-1'''-yl-méthyl)-phénoxy]-propyl}-carbothioamide,
1-{5-[amino]-3-[4'-(méthyl)-pipérazinyl]-1H-1,2,4-triazol-1-yl}-N-{3'-[3''-(pipéridine-1'''-yl-méthyl)-phénoxy]-propyl}-carbothioamide et
1-{5-[amino]-3-[méthylthio]-1H-1,2,4-triazol-1-yl}-N-{2'-[3'', 4''-di(méthoxy)-phényl]-éthyl}-carbothioamide.

**10.** Procédé de préparation des dérivés de 5-(amino)-triazolylcarbothioamides selon la revendication 1 ou l'une des revendications 3 à 9, ou des dérivés de 5-(amino)-triazolylcarbothioamides utilisés selon la revendication 2, caractérisé en ce qu'on fait réagir des triazolyldithioesters répondant à la formule générale :

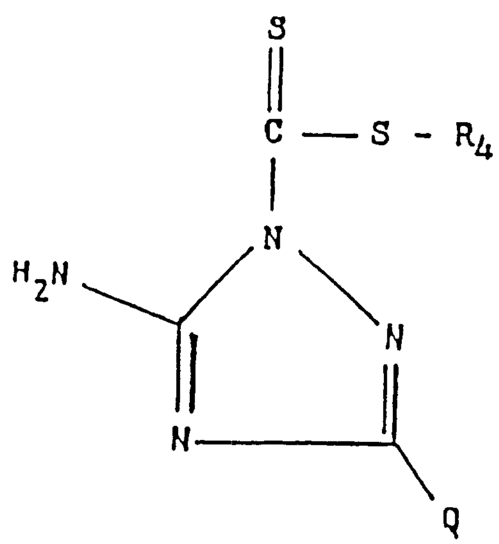

II

dans laquelle

$R_4$ représente un reste alkyle ayant de 1 à 4 atomes de carbone ou un reste phénylalkyle, éventuellement substitué par un atome d'halogène, ayant de 1 à 4 atomes de carbone dans la partie alkyle, et

Q a les significations indiquées dans les revendications 1 à 8,

avec des amines répondant à la formule générale :

$Y—NH_2$ III,

et ensuite, on transforme éventuellement, d une manière connue en soi, les dérivés de 5-(amino)-triazolylcarbothioamides obtenus, répondant à la formule générale I, en sels d'addition d'acides, et on transforme éventuellement les sels d'addition d'acides obtenus à partir des dérivés de 5-(amino)-triazolylcarbothioamides de formule générale I en dérivés 5-(amino)-triazolylcarbothioamides libres, répondant à la formule générale I, ou en d'autres sels d'addition d'acides.

**Revendications pour les Etats contractants suivants : ES, GR**

**1.** Procédé de préparation de dérivés de 5-(amino)-triazolylcarbothioamides répondant à la formule générale :

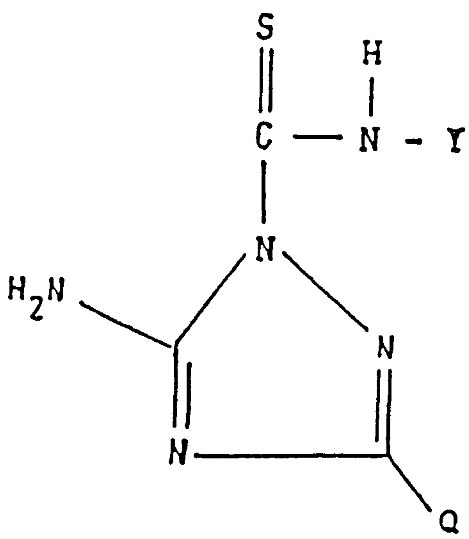

I

dans lesquelles

Q représente un atome d'hydrogène, un reste hétérocyclique éventuellement substitué par 1 ou plusieurs restes alkyles linéaires et/ou ramifiés, ayant de 1 à 4 atomes de carbone, comportant de 4 à 8 chaînons, présentant 1 ou plusieurs atomes d'azote et/ou d'oxygène, ayant un caractère aromatique ou étant saturé d'une manière quelconque, ou un reste répondant à la formule :

$- S - R_1$ IV

ou

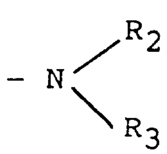

V

dans laquelle

$R_1$ représente un reste alkyle linéaire ou ramifié comportant de 1 à 6 atomes de carbone, et

$R_2$ et $R_3$ signifient, indépendamment l'un de l'autre, un atome d'hydrogène ou des restes alkyles linéaires ou ramifiés comportant de 1 à 4 atomes de carbone, ou des restes alkényles linéaires ou ramifies comportant de 2 à 6 atomes de carbone, et

Y signifie un reste alkyle comportant de 1 à 4 atomes de carbone, éventuellement substitué par 1 ou plusieurs groupes hydroxy ou par un ou plusieurs restes alcoxy

linéaires et/ou ramifiés comportant de 1 à 4 atomes de carbone, un reste phénylalkyle comportant de 1 à 4 atomes de carbone dans la partie alkyle linéaire ou ramifié, éventuellement substitué dans le cycle aromatique par 1 ou plusieurs restes alcoxy linéaires et/ou ramifiés comportant de 1 à 4 atomes de carbone, ou un reste phénoxyalkyle comportant de 1 à 4 atomes de carbone dans la partie alkyle linéaire ou ramifié, substitué dans le cycle phénylique par un reste alkyle linéaire ou ramifié comportant de 1 à 4 atomes de carbone, éventuellement substitué par un reste hétérocyclique comportant un atome d'azote, ayant de 4 à 8 chaînons, présentant un caractère aromatique ou étant saturé d'une manière quelconque,

ainsi que leurs sels d'addition d'acides,
caractérisé en ce qu'on fait réagir des triazolyldithioesters répondant à la formule générale :

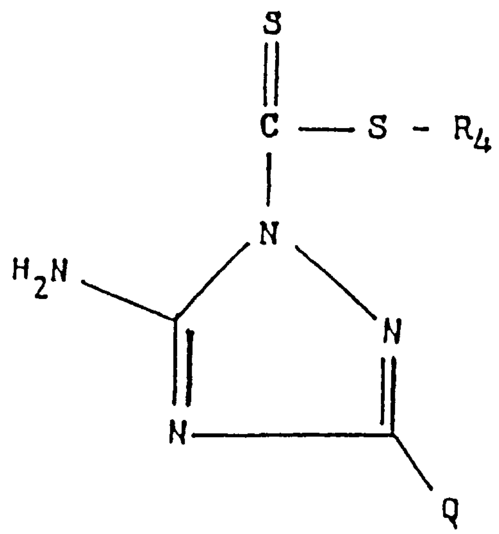

II

dans laquelle

$R_4$ représente un reste alkyle ayant de 1 à 4 atomes de carbone ou un reste phénylalkyle, éventuellement substitué par un atome d'halogène, ayant de 1 à 4 atomes de carbone dans la partie alkyle, et

Q a les significations indiquées dans les revendications 1 à 6,

avec des amines répondant à la formule générale :

$Y{-}NH_2$ III,

et ensuite, on transforme éventuellement, d'une manière connue en soi, les dérivés de 5-(amino)-triazolylcarbothioamides obtenus, répondant à la formule générale I, en sels d'addition d'acides, et on transforme éventuellement les sels d'addition d'acides obtenus à partir des dérivés de 5-(amino)-triazolylcarbothioamides de formule générale I en dérivés 5-(amino)-triazolylcarbothioamides libres, répondant à la formule générale I, ou en d'autres sels d'addition d'acides.

**2.** Procédé selon la revendication 1, caractérisé en ce qu'on prépare des dérivés de 5-(amino)-triazolylcarbothioamides dans lesquels le ou les restes alkyles au moyen duquel/desquels le reste hétérocyclique peut être substitué, qui peut être représenté par Q, le ou les restes alkyles qui peuvent être représentés par $R_2$ et/ou $R_3$, le ou les restes alcoxy, au moyen duquel/desquels le reste alkyle est substitué, qui peut être représenté par Y, la partie alkyle du reste phénylalkyle, qui peut être représentée par Y, le ou les restes alcoxy, au moyen duquel/desquels le reste phénylalkyle est substitué, qui peut être représenté par Y, ou le reste alkyle au moyen duquel le reste phénoxyalkyle peut être substitué, qui peut être représenté par Y, comporte(nt) 1 ou 2 atomes de carbone, ou la partie alkyle du reste phénoxyalkyle, qui peut être représentée par Y, ou le reste alkyle pouvant être représenté par Y, sont des restes alkyles comportant de 2 à 4, en particulier 2 ou 3 atomes de carbone.

**3.** Procédé selon la revendication 1 ou 2, caractérisé en ce qu'on prépare des dérivés de 5-(amino)-triazolylcarbothioamides dans lesquels le reste alkyle, qui peut être représenté par $R_1$, comporte de 1 à 4, en particulier 1 ou 2 atomes de carbone.

**4.** Procédé selon l'une des revendications 1 à 3, caractérisé en ce qu'on prépare des dérivés de 5-(amino)-triazolylcarbothioamides dans lesquels le ou les restes alkényles, qui peuvent être représentés par $R_2$ et/ou $R_3$, comportent de 2 à 4, en particulier 2 ou 3 atomes de carbone.

**5.** Procédé selon l'une des revendications 1 à 4, caractérisé en ce qu'on prépare des dérivés de 5-(amino)-triazolylcarbothioamides dans lesquels le reste hétérocyclique, qui peut être représenté par Q, et/ou le reste hétérocyclique qui peut être un substituant pour le reste alkyle qui peut être un substituant pour le reste phénoxyalkyle qui peut être représenté par Y, est un reste à 5 ou 6 chaînons, en particulier un reste pipéridyle, morpholinyle, pipérazinyle, furyle, imidazolyle, pyridyle, pyrimidinyle, pyrrolyle, pyrazolyle, pyridazinyle, isoxazolyle, pyrrolinyle, pyrrolidinyle, imidazolidinyle, imidazolinyle, pyrazolidinyle, pyrazolinyle, pyranyle ou $\delta$-3-pipéridine-1-yle.

**6.** Procédé selon l'une des revendications 1 à 3 ou selon la revendication 5, caractérisé en ce qu'on utilise des dérivés de 5-(amino)-triazolylcarbothioamides dans lesquels Q signifie un reste morpholinyle, un reste di-(alkyl)-amino, un reste alkylthio ou un reste 4-(méthyl)-pipérazinyle.

**7.** Procédé selon l'une des revendications 1 à 6, caractérisé en ce qu'on prépare des dérivés de 5-(amino)-triazolylcarbothioamides dans lesquels Y signifie un reste 3-[3'-(pipéridine-1''-ylméthyl)-phénoxy]-propyle ou un reste 2-(phényl)-éthyle substitué dans le cycle aromatique par 1 ou 2 restes alcoxy, en particulier le reste 2-[3',4'-di-(méthoxy)-phényl]-éthyle.

**8.** Procédé selon l'une des revendications 1 à 3 ou 5 à 7, caractérisé en ce qu'on prépare les dérivés suivants de 5-(amino)-triazolylcarbothioamides :
1-{5-[amino]-3-[morpholino]-1H-1,2,4-triazol-1-yl}-N-{3'-[3''-(pipéridine-1'''-yl-méthyl)-phénoxy]-propyl}-carbothioamide,
1-{5-[amino]-3-[diméthylamino]-1H-1,2,4-triazol-1-yl}-N-{3'-[3''-(pipéridine-1'''-yl-méthyl)-phénoxy]-propyl}-carbothioamide,
1-{5-[amino]-3-[4'-(méthyl)-pipérazinyl]-1H-1,2,4-triazol-1-yl}-N-{3'-[3''-(pipéridine-1'''-yl-méthyl)-phénoxy]-propyl}-carbothioamide ou
1-{5-[amino]-3-[méthylthio]-1H-1,2,4-triazol-1-yl}-N-{2'-[3'',4''-di-(méthoxy)-phényl]-éthyl}-carbothioamide.